(19) Europäisches Patentamt / European Patent Office / Office européen des brevets

(11) **EP 3 808 882 B1**

(12) **EUROPEAN PATENT SPECIFICATION**

(45) Date of publication and mention
of the grant of the patent:
**30.04.2025 Bulletin 2025/18**

(21) Application number: **19782230.7**

(22) Date of filing: **03.04.2019**

(51) International Patent Classification (IPC):
*D02G 3/04* ^(2006.01)    *D01F 4/02* ^(2006.01)
*D03D 15/00* ^(2021.01)    *D04B 1/14* ^(2006.01)
*D06M 11/01* ^(2006.01)    *C07K 14/435* ^(2006.01)
*C12N 15/09* ^(2006.01)    *C12N 15/12* ^(2006.01)

(52) Cooperative Patent Classification (CPC):
**D03D 15/233; C07K 14/43518; D02G 3/045;
D03D 15/225; D03D 15/41; D03D 15/47;
D04B 1/14; D06M 11/01;** C12N 15/09; D01D 5/06;
D01F 4/02; D10B 2201/20; D10B 2211/22

(86) International application number:
**PCT/JP2019/014892**

(87) International publication number:
**WO 2019/194258 (10.10.2019 Gazette 2019/41)**

(54) **BLENDED YARN, KNITTED/WOVEN BODY OF SAME, AND METHOD FOR MANUFACTURING SAID KNITTED/WOVEN BODY**

MISCHGARN, STRICK-/WEBSTOFF DARAUS UND VERFAHREN ZUR HERSTELLUNG DES STRICK-/WEBSTOFFES

FIL MÉLANGÉ, CORPS TRICOTÉ/TISSÉ DE CELUI-CI ET PROCÉDÉ DE FABRICATION DUDIT CORPS TRICOTÉ/TISSÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.04.2018 JP 2018071886
28.09.2018 JP 2018185166**

(43) Date of publication of application:
**21.04.2021 Bulletin 2021/16**

(73) Proprietors:
• **Hasetora Spinning Co., Ltd.**
**Gifu 501-6236 (JP)**
• **Spiber Inc.**
**Tsuruoka-shi, Yamagata 997-0052 (JP)**

(72) Inventors:
• **ASANO Tetsuhiro**
**Hashima-shi, Gifu 501-6257 (JP)**
• **OZEKI Akihiko**
**Tsuruoka-shi, Yamagata 997-0052 (JP)**

(74) Representative: **Handley, Matthew Edward et al**
**Venner Shipley LLP**
**St James's**
**79 Oxford Street**
**Manchester M1 6EG (GB)**

(56) References cited:
**WO-A1-2012/165477    WO-A2-2006/008163
CN-A- 102 877 176    CN-A- 103 132 198
CN-A- 103 349 383    CN-A- 104 178 898
CN-U- 205 416 602    JP-A- 2004 532 362
JP-A- 2014 198 041    JP-A- H01 213 427
JP-A- H09 310 235**

Remarks:
The complete document including Reference Table(s) and the Sequence Listing(s) can be downloaded from the EPO website

EP 3 808 882 B1

## Description

### Technical Field

The present invention relates to a blended yarn, a knitted or woven body of the same, and a method for manufacturing the knitted or woven body.

### Background Art

Fibers such as rayon, cupra, and lyocell, which are regenerated cellulose fibers, have a soft texture, and thus, they are knitted or woven into a knitted or woven body, which is used in various applications such as clothing (see, for example, JP-B-6162566 ). The production of different fibres and fabrics is disclosed, for example, in CN104178898, CN103349383, and Mittal et al. ACS Nano 2017, 11, 5148-5159. WO-A-2006/008163 describes recombinant spider silk proteins, which can be used for spinning filaments. CN-A-103132198 discloses a blended yarn comprising silkworm chrysalis protein fibre and tencel. WO-A-2012/165477 describes a protein fibre containing silk fibroin.

### Summary of Invention

### Technical Problem

The present inventors have conducted various studies on a knitted or woven body for which regenerated cellulose fibers are used, and found that upon wetting with water and drying, the tension and the stiffness of the knitted or woven body of the regenerated cellulose fiber are decreased in some cases. In order to prevent such a decrease in the tension and the stiffness of the knitted or woven body of the regenerated cellulose fibers, it is conceivable, for example, to apply a general waterproof finish such as water-proofing or water-repellent coating to the knitted or woven body. However, in such a case, the soft texture which the regenerated cellulose fibers have may be impaired.

The present invention aims at providing a blended yarn with which a knitted or woven body, wherein a decrease in tension and stiffness due to moistness is reduced, can be manufactured, even in a case where the blended yarn is used for the manufacture of the knitted or woven body; a knitted or woven body of the same; and a method for manufacturing the knitted or woven body.

### Solution to Problem

The present invention is defined in the claims. The technical disclosure set out below may in some respects go beyond the scope of the claimed invention, and elements of the disclosure which do not fall within the scope of the claims are provided for information, to place the actual invention claimed in a broader technical context.

### Advantageous Effects of Invention

According to the blended yarn and the method according to the present invention, it is possible to provide a knitted or woven body wherein a decrease in tension and stiffness due to moistness is reduced. Therefore, according to the present invention, it is expected that it becomes possible to control a decrease in the drape property of the knitted or woven body due to the decrease in tension and stiffness. In addition, according to the present invention, it is possible to maintain an excellent texture unique to a regenerated cellulose fiber as much as possible.

### Brief Description of Drawings

Fig. 1 is a schematic diagram illustrating an example of a domain sequence of a modified fibroin.
Fig. 2 is a diagram illustrating a distribution of z/w (%) values of a naturally occurring fibroin.
Fig. 3 is a diagram illustrating a distribution of x/y (%) values of a naturally occurring fibroin.
Fig. 4 is a schematic diagram illustrating an example of a domain sequence of a modified fibroin.
Fig. 5 is a schematic diagram illustrating an example of a domain sequence of a modified fibroin.
Fig. 6 is an explanatory diagram schematically illustrating an example of a spinning device for manufacturing a modified fibroin filament.
Fig. 7 is a photograph of a knitted fabric in Examples.
Fig. 8 is a photograph of a knitted fabric in Comparative Examples.

Fig. 9 is a graph illustrating an example of the results of a hygroscopic heat generation test.

**Description of Embodiments**

<Blended Yarn>

One aspect of the invention relates to a blended yarn containing a modified fibroin fiber and a regenerated cellulose fiber. The modified fibroin fiber contains a modified fibroin which will be described later as a main component, and may be a filament (long fiber) or may be a staple (short fiber). The modified fibroin fiber may contain components other than the modified fibroin and impurities, such as proteins other than the modified fibroin. In the present invention, since the modified fibroin fiber having a characteristics of shrinking upon drying after moistening is contained in the blended yarn, it is considered that when a knitted or woven body formed using such a blended yarn is moistened and dried, the knitting density, the weaving density, and the like are increased due to the shrinkage of the modified fibroin fiber, and as a result, the above-described effects can be exhibited.

The modified fibroin fiber may have a shrinkage rate, which will be described later, of more than 7%, 15% or more, 25% or more, 32% or more, 40% or more, 48% or more, 56% or more, 64% or more, or 72% or more, when brought into contact with an aqueous medium. The shrinkage rate is usually 80% or less. Here, the shrinkage rate of the modified fibroin fiber is defined by the following formula:

Shrinkage rate = {1 - (Length of modified fibroin fiber after being brought into contact with aqueous medium/Length of modified fibroin fiber before being brought into contact with aqueous medium)} $\times$ 100 (%).

The modified fibroin fiber may be a modified fibroin crimped fiber having a crimp. The degree of crimping is not limited, and the number of crimps may be, for example, 10 to 100 crimps/40 mm, or 20 to 40 crimps/40 mm.

The modified fibroin fiber preferably has an excellent flame retardancy, and may have a limiting oxygen index (LOI) value of 18 or more, 20 or more, 22 or more, 24 or more, 26 or more, 28 or more, 29 or more, or 30 or more. In the present specification, the LOI value is a value measured in accordance with "Test Method for Granular or Low-Melting-Point Synthetic Resin" described in "Notice No. 50 of the Office of Hazardous Materials Regulation (as of May 31, 1995)".

In order to exert sufficient flame retardancy, the modified fibroin fiber may contain a hydrophilic modified fibroin and/or may contain a modified fibroin having an average value of the hydropathy index (average HI) of 0 or less.

The modified fibroin fiber preferably has an excellent hygroscopic exothermicity, and may have a maximum hygroscopic heat generation, as determined according to the following Formula A, of more than 0.025°C/g. Here, the sample in Formula A refers to a modified fibroin fiber.

Formula A: Maximum hygroscopic heat generation = {(Highest temperature of a sample when the sample has been transferred to a high humidity environment after being placed in a low humidity environment until a temperature of the sample reaches equilibrium) - (Temperature of the sample when the sample is being transferred to the high humidity environment after being placed in the low humidity environment until the temperature of the sample reaches equilibrium)} (°C) / Sample weight (g) [in Formula A, the low humidity environment means an environment of a temperature of 20°C and a relative humidity of 40%, and the high humidity environment means an environment of a temperature of 20°C and a relative humidity of 90%].

The maximum hygroscopic heat generation of the modified fibroin fiber may be 0.026°C/g or more, 0.027°C/g or more, 0.028°C/g or more, 0.029°C/g or more, 0.030°C/g or more, 0.035°C/g or more, or 0.040°C/g or more. The upper limit of the maximum hygroscopic heat generation is not particularly limited, but is usually 0.060°C/g or less.

In order to exert a sufficient hygroscopic exothermicity, the modified fibroin fiber may contain a hydrophobic modified fibroin and/or may contain a modified fibroin having an average value of the hydropathy index (average HI) of more than 0.

The modified fibroin fiber according to the present embodiment preferably has an excellent heat retention, and may have a heat retention index determined according to the following Formula B of 0.20 or more.

Heat retention index = Heat retention rate (%) / Basis weight (g/m$^2$) of sample                    Formula B:

Here, in the present specification, the heat retention rate means a heat retention rate measured by a dry contact method using a Thermo Labo II type tester (under a wind of 30 cm/sec), and is a value measured by the method described in Reference Examples which will be described later.

The heat retention index of the modified fibroin fiber according to the present embodiment may be 0.22 or more, may be 0.24 or more, may be 0.26 or more, may be 0.28 or more, may be 0.30 or more, and may be 0.32 or more. The upper limit of the heat retention index is not particularly limited, but may be, for example, 0.60 or less, or 0.40 or less.

The regenerated cellulose fiber is not particularly limited, and may be a known regenerated cellulose fiber such as rayon, cupra, lyocell, and acetate. Among the regenerated cellulose fibers, the lyocell fiber is preferable from the viewpoint that it has a softer texture and a higher strength. The regenerated cellulose fiber may be a filament or may be a staple. The regenerated cellulose fiber may be crosslinked with a crosslinking agent such as a compound or resin that reacts with a hydroxy group in a molecule of the regenerated cellulose fiber.

The regenerated cellulose fiber may be a regenerated cellulose crimped fiber having a crimp. The degree of crimping is not limited, and the number of crimps may be, for example, 10 to 100 crimps/40 mm, or 20 to 40 crimps/40 mm. The regenerated cellulose fiber is preferably a lyocell crimped fiber.

The blended yarn may consist only of the modified fibroin fiber and the regenerated cellulose fiber, or may also contain another fiber other than the modified fibroin fiber and the regenerated cellulose fiber. Such another fiber may be a natural fiber or may be a chemical fiber.

The amount of the modified fibroin fiber and the amount of the regenerated cellulose fiber in the blended yarn are not particularly limited, but from the viewpoint of preventing or suppressing a decrease in tension and stiffness due to moistening of the knitted or woven body, the amount of the modified fibroin fiber in the blended yarn may be, for example, 5% to 90% by mass, 5% to 40% by mass, 10% to 70% by mass, 10% to 50% by mass, 20% to 40% by mass, 20% to 30% by mass, or 15% to 25% by mass. Furthermore, from the viewpoint of maintaining an excellent texture unique to the regenerated cellulose fiber, the amount of the regenerated cellulose fiber in the blended yarn may be, for example, 60% to 95% by mass, 70% to 90% by mass, 70% to 85% by mass, or 75% to 85% by mass. The amount of the another fiber in the blended yarn may be, for example, 5% to 30% by mass, 5% to 20% by mass, 5% to 10% by mass, or 5% to 15% by mass.

<Modified Fibroin>

The modified fibroin according to the present embodiment is a protein containing a domain sequence represented by Formula 1: $[(A)_n \text{ motif-REP}]_m$ or Formula 2: $[(A)_n \text{ motif-REP}]_m\text{-}(A)_n$ motif. In the modified fibroin, an amino acid sequence (an N-terminal sequence and a C-terminal sequence) may be further added to either or both of the N-terminal side and the C-terminal side of the domain sequence. The N-terminal sequence and the C-terminal sequence are typically, but not limited to, regions that do not have a repetition of amino acid motifs characteristic of a fibroin, and that consist of amino acids of about 100 residues.

The "modified fibroin" in the present specification means an artificially produced fibroin (an artificial fibroin). The modified fibroin is a fibroin in which the domain sequence is different from an amino acid sequence of a naturally occurring fibroin. The "naturally occurring fibroin" referred to in the present specification also is a protein containing a domain sequence represented by Formula 1: $[(A)_n \text{ motif-REP}]_m$ or Formula 2: $[(A)_n \text{ motif-REP}]_m\text{-}(A)_n$ motif.

The "modified fibroin" may be modified fibroin whose amino acid sequence has been modified based on the amino acid sequence of a naturally occurring fibroin (for example, a modified fibroin whose amino acid sequence has been modified by altering a cloned gene sequence of a naturally occurring fibroin), or may be a modified fibroin that is artificially designed and synthesized independently of a naturally occurring fibroin (for example, a modified fibroin having a desired amino acid sequence by chemically synthesizing a nucleic acid encoding a designed amino acid sequence).

The "domain sequence" in the present specification is an amino acid sequence that produces a crystalline region (typically corresponding to an $(A)_n$ motif of an amino acid sequence) and an amorphous region (typically corresponding to REP of an amino acid sequence) unique to a fibroin, and refers to an amino acid sequence represented by Formula 1: $[(A)_n \text{ motif-REP}]_m$ or Formula 2: $[(A)_n \text{ motif-REP}]_m\text{-}(A)_n$ motif. Here, the $(A)_n$ motif represents an amino acid sequence mainly containing alanine residues, and the number of amino acid residues in the $(A)_n$ motif is 2 to 27. The number of amino acid residues in the $(A)_n$ motif may be an integer of 2 to 20, 4 to 27, 4 to 20, 8 to 20, 10 to 20, 4 to 16, 8 to 16, or 10 to 16. Further, the proportion of the number of alanine residues with respect to the total number of amino acid residues in the $(A)_n$ motif is 40% or more, and may be 60% or more, 70% or more, 80% or more, 83% or more, 85% or more, 86% or more, 90% or more, 95% or more, or 100% (meaning that the $(A)_n$ motif is composed of only alanine residues). Among a plurality of $(A)_n$ motifs present in the domain sequence, at least seven of the $(A)_n$ motifs may be composed only of alanine residues. REP represents an amino acid sequence composed of 2 to 200 amino acid residues. REP may be an amino acid sequence composed of 10 to 200 amino acid residues, and may be an amino acid sequence composed of 10 to 40, 10 to 60, 10 to 80, 10 to 100, 10 to 120, 10 to 140, 10 to 160, or 10 to 180 amino acid residues. m represents an integer of 2 to 300, and may be an integer of 8 to 300, 10 to 300, 20 to 300, 40 to 300, 60 to 300, 80 to 300, 100 to 300, 10 to 200, 20 to 200, 20 to 180, 20 to 160, 20 to 140, or 20 to 120. A plurality of $(A)_n$ motifs may have the same amino acid sequence or amino acid sequences different from each other. A plurality of REPs may have the same amino acid sequence or amino acid sequences different from each other.

The modified fibroin according to the present embodiment may be obtained, for example, by subjecting a cloned gene sequence of a naturally occurring fibroin to a modification of an amino acid sequence corresponding to, for example, substitution, deletion, insertion, and/or addition of one or a plurality of amino acid residues. The substitution, deletion, insertion, and/or addition of an amino acid residue may be performed by methods well known to those skilled in the art, such as site-directed mutagenesis. Specifically, the modification may be performed in accordance with the method described in literature such as Nucleic Acid Res. 10, 6487 (1982) and Methods in Enzymology, 100, 448 (1983).

A naturally occurring fibroin is a protein containing a domain sequence represented by Formula 1: $[(A)_n$ motif-REP$]_m$ or Formula 2: $[(A)_n$ motif-REP$]_m$-$(A)_n$ motif, and specific examples thereof include a fibroin produced by insects or spiders.

Examples of the fibroin produced by insects include silk proteins produced by silkworms such as Bombyx mori, Bombyx mandarina, Antheraea yamamai, Anteraea pernyi, Eriogyna pyretorum, Pilosamia Cynthia ricini, Samia cynthia, Caligura japonica, Antheraea mylitta, and Antheraea assama; and hornet silk proteins discharged by larvae of Vespa simillima xanthoptera.

More specific examples of the fibroin produced by insects include a silkworm fibroin L chain (GenBank Accession No. M76430 (nucleotide sequence), and AAA27840.1 (amino acid sequence)).

Examples of the fibroin produced by spiders include spider silk proteins produced by spiders belonging to the genus Araneus such as Araneus ventricosus, Araneus diadematus, Araneus pinguis, Araneus pentagrammicus and Araneus nojimai, spiders belonging to the genus Neoscona such as Neoscona scylla, Neoscona nautica, Neoscona adianta and Neoscona scylloides, spiders belonging to the genus Pronus such as Pronous minutes, spiders belonging to the genus Cyrtarachne such as Cyrtarachne bufo and Cyrtarachne inaequalis, spiders belonging to the genus Gasteracantha such as Gasteracantha kuhli and Gasteracantha mammosa, spiders belonging to the genus Ordgarius such as Ordgarius hobsoni and Ordgarius sexspinosus, spiders belonging to the genus Argiope such as Argiope amoena, Argiope minuta and Argiope bruennich, spiders belonging to the genus Arachnura such as Arachnura logio, spiders belonging to the genus Acusilas such as Acusilas coccineus, spiders belonging to the genus Cytophora such as Cytophora moluccensis, Cytophora exanthematica and Cytophora unicolor, spiders belonging to the genus Poltys such as Poltys illepidus, spiders belonging to the genus Cyclosa such as Cyclosa octotuberculata, Cyclosa sedeculata, Cyclosa vallata and Cyclosa atrata, and spiders belonging to the genus Chorizopes such as Chorizopes nipponicus; and spider silk proteins produced by spiders belonging to the genus Tetragnatha such as Tetragnatha praedonia, Tetragnatha maxillosa, Tetragnatha extensa and Tetragnatha squamata, spiders belonging to the genus Leucauge such as Leucauge magnifica, Leucauge blanda and Leucauge subblanda, spiders belonging to the genus Nephila such as Nephila clavata and Nephila pilipes, spiders belonging to the genus Menosira such as Menosira ornata, spiders belonging to the genus Dyschiriognatha such as Dyschiriognatha tenera, spiders belonging to the genus Latrodectus such as Latrodectus mactans, Latrodectus hasseltii, Latrodectus geometricus and Latrodectus tredecimguttatus, and spiders belonging to the family Tetragnathidae such as spiders belonging to the genus Euprosthenops. Examples of the spider silk proteins include traction yarn proteins such as MaSp (MaSp1 and MaSp2) and ADF (ADF3 and ADF4), and MiSp (MiSp1 and MiSp2).

More specific examples of the spider silk protein produced by spiders include fibroin-3 (adf-3) [derived from Araneus diadematus] (GenBank Accession No. AAC47010 (amino acid sequence), U47855 (nucleotide sequence)), fibroin-4 (adf-4) [derived from Araneus diadematus] (GenBank Accession No. AAC47011 (amino acid sequence), U47856 (nucleotide sequence)), dragline silk protein spidroin 1 [derived from Nephila clavipes] (GenBank Accession No. AAC04504 (amino acid sequence), U37520 (nucleotide sequence)), major ampullate spidroin 1 [derived from Latrodectus hesperus] (GenBank Accession No. ABR68856 (amino acid sequence), EF595246 (nucleotide sequence)), dragline silk protein spidroin 2 [derived from Nephila clavata] (GenBank Accession No. AAL32472 (amino acid sequence), AF441245 (nucleotide sequence)), major ampullate spidroin 1 [derived from Euprosthenops australis] (GenBank Accession No. CAJ00428 (amino acid sequence), AJ973155 (nucleotide sequence)) and major ampullate spidroin 2 [Euprosthenops australis] (GenBank Accession No. CAM32249.1 (amino acid sequence), AM490169 (nucleotide sequence)), minor ampullate silk protein 1 [Nephila clavipes] (GenBank Accession No. AAC14589.1 (amino acid sequence), minor ampullate silk protein 2 [Nephila clavipes] (GenBank Accession No. AAC14591.1 (amino acid sequence)), and minor ampullate spidroin-like protein [Nephilengys cruentata] (GenBank Accession No. ABR37278.1 (amino acid sequence)).

More specific examples of the naturally occurring fibroin further include a fibroin whose sequence information is registered in NCBI GenBank. For example, sequences thereof may be confirmed by extracting sequences that has a keyword of spidroin, ampullate, fibroin, "silk and polypeptide", or "silk and protein" in DEFINITION, among sequences containing INV as DIVISION in sequence information registered in NCBI GenBank; sequences that have a specific character string of a product from CDS; or sequences that have a specific character string described for TISSUE TYPE from SOURCE.

The modified fibroin according to the present embodiment may be a modified silk fibroin (a modified fibroin obtained by modifying an amino acid sequence of a silk protein produced by silkworm), or may be a modified spider silk fibroin (a modified fibroin obtained by modifying an amino acid sequence of a spider silk protein produced by spiders). As the modified fibroin, a modified spider silk fibroin is preferable. The modified spider silk fibroin is also excellent in heat retention, hygroscopic exothermicity, and/or flame retardancy.

Specific examples of the modified fibroin include a modified fibroin derived from a large spinal canal thread protein produced in the major ampullate gland of a spider (first modified fibroin), a modified fibroin having a domain sequence with a reduced content of glycine residues (second modified fibroin), a modified fibroin having a domain sequence with a reduced content of $(A)_n$ motifs (third modified fibroin), a modified fibroin with a reduced content of glycine residues and $(A)_n$ motifs (fourth modified fibroin), a modified fibroin having a domain sequence containing a region having a locally high hydropathy index (fifth modified fibroin), and a modified fibroin having a domain sequence with a reduced content of glutamine residues (sixth modified fibroin).

Examples of the first modified fibroin include a protein containing a domain sequence represented by Formula 1: $[(A)_n$ motif- $REP]_m$. In the first modified fibroin, the number of amino acid residues of the $(A)_n$ motif is preferably an integer of 3 to 20, more preferably an integer of 4 to 20, still more preferably an integer of 8 to 20, even still more preferably an integer of 10 to 20, further still preferably an integer of 4 to 16, particularly preferably an integer of 8 to 16, and most preferably an integer of 10 to 16. In the first modified fibroin, the number of amino acid residues constituting REP in Formula 1 is preferably 10 to 200 residues, more preferably 10 to 150 residues, still more preferably 20 to 100 residues, and even still more preferably 20 to 75 residues. In the first modified fibroin, the total number of the glycine residues, the serine residues, and the alanine residues contained in the amino acid sequence represented by Formula 1: $[(A)_n$ motif-$REP]_m$ is preferably 40% or more, more preferably 60% or more, and still more preferably 70% or more with respect to the total number of amino acid residues.

The first modified fibroin may be a polypeptide that contains a unit of an amino acid sequence represented by Formula 1: $[(A)_n$ motif-$REP]_m$, and has the amino acid sequence set forth in any of SEQ ID NOs: 1 to 3 or an amino acid sequence having 90% or more homology with the amino acid sequence set forth in any of SEQ ID NOs: 1 to 3 as the C-terminal sequence.

The amino acid sequence set forth in SEQ ID NO: 1 is the same as the amino acid sequence consisting of 50 amino acid residues at the C-terminal of the amino acid sequence of ADF3 (GI: 1263287, NCBI), the amino acid sequence set forth in SEQ ID NO: 2 is the same as the amino acid sequence obtained by removing 20 residues from the C-terminal of the amino acid sequence set forth in SEQ ID NO: 1, and the amino acid sequence set forth in SEQ ID NO: 3 is the same as the amino acid sequence obtained by removing 29 residues from the C-terminal of the amino acid sequence set forth in SEQ ID NO: 1.

More specific examples of the first modified fibroin include a modified fibroin containing (1-i) the amino acid sequence set forth in SEQ ID NO: 4 (recombinant spider silk protein ADF3KaiLargeNRSH1), or (1-ii) an amino acid sequence having 90% or more sequence identity with the amino acid sequence set forth in SEQ ID NO: 4. The sequence identity is preferably 95% or more.

The amino acid sequence shown in SEQ ID NO 4 is obtained by performing mutation on the amino acid sequence of ADF3 in which an amino acid sequence (SEQ ID NO 5) consisting of a start codon, His10 tag, and HRV3C protease (Human rhinovirus 3C protease) recognition site is added to the N-terminal, such that the 1st to 13th repeat regions are approximately doubled, and the translation terminates at the 1154th amino acid residue. The amino acid sequence of the C-terminal of the amino acid sequence shown in SEQ ID NO 4 is the same as the amino acid sequence shown in SEQ ID NO 3.

The modified fibroin (1-i) may be a modified fibroin consisting of the amino acid sequence set forth in SEQ ID NO: 4.

The second modified fibroin has a domain sequence that has an amino acid sequence with a reduced content of glycine residues, as compared with a naturally occurring fibroin. It can be said that the second modified fibroin is a modified fibroin having an amino acid sequence that corresponds to an amino acid sequence in which, at least one or a plurality of glycine residues in REP are substituted with other amino acid residues, as compared with a naturally occurring fibroin.

The second modified fibroin may have a domain sequence that has an amino acid sequence corresponding to an amino acid sequence in which, in at least one motif sequence selected from GGX and GPGXX (where G represents a glycine residue, P represents a proline residue, and X represents an amino acid residue other than glycine) in REP, at least one glycine residue in one or a plurality of motif sequences is substituted with another amino acid residue, as compared with a naturally occurring fibroin.

In the second modified fibroin, the proportion of the above-described motif sequences in which a glycine residue is substituted with another amino acid residue may be 10% or more with respect to the all motif sequences.

The second modified fibroin may be a modified fibroin that contains a domain sequence represented by Formula 1: $[(A)_n$ motif-$REP]_m$, that has an amino acid sequence in which z/w is 30% or more, 40% or more, 50% or more, or 50.9% or more, where z is the total number of amino acid residues of an amino acid sequence consisting of XGX (where X represents an amino acid residue other than glycine) contained in all REPs in the domain sequence excluding the sequence between the $(A)_n$ motif positioned closest to the C-terminal side and the C-terminal of the domain sequence; and where w is the total number of amino acid residues in the domain sequence excluding the sequence between the $(A)_n$ motif positioned closest to the C-terminal side and the C-terminal of the domain sequence. The number of alanine residues with respect to a total number of amino acid residues in the $(A)_n$ motif may be 83% or more, but is preferably 86% or more, more preferably 90% or more, still more preferably 95% or more, and even still more preferably 100% (which means that the $(A)_n$ motif consists only of alanine residues).

The second modified fibroin is preferably a modified fibroin in which one glycine residue of the GGX motif is substituted with another amino acid residue to increase the content ratio of the amino acid sequence consisting of XGX. In the second modified fibroin, the content ratio of the amino acid sequence consisting of GGX in the domain sequence is preferably 30% or less, more preferably 20% or less, still more preferably 10% or less, even still more preferably 6 % or less, further still more preferably 4% or less, and particularly preferably 2% or less. The content ratio of the amino acid sequence consisting of GGX in the domain sequence can be calculated by the same method as the method for calculating a content ratio (z/w) of an amino acid sequence consisting of XGX below.

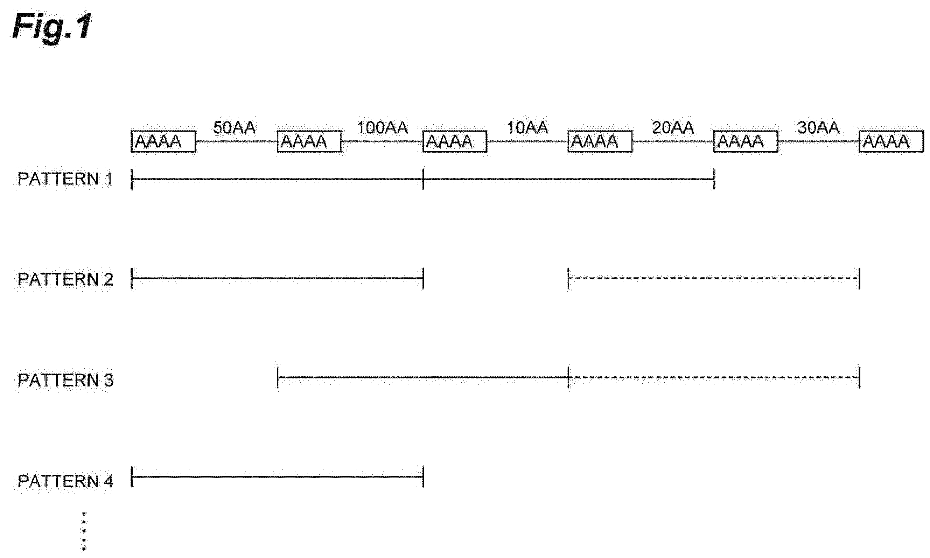

The method for calculating z/w will be described in more detail. First, in a fibroin (a modified fibroin or a naturally occurring fibroin) containing a domain sequence represented by Formula 1: $[(A)_n$ motif-REP$]_m$, an amino acid sequence consisting of XGX is extracted from all REPs contained in the domain sequence excluding the sequence between the $(A)_n$ motif positioned closest to the C-terminal side and the C-terminal of the domain sequence. The total number of amino acid residues constituting XGX is z. For example, in a case where 50 amino acid sequences consisting of XGX (without an overlap) are extracted, z is $50 \times 3 = 150$. Further, for example, in a case where there is X contained in two XGXs (X at the center), as in the case of an amino acid sequence consisting of XGXGX, the calculation is performed by deducting the overlap (in the case of XGXGX, 5 amino acid residues). w is the total number of amino acid residues contained in the domain sequence excluding the sequence between the $(A)_n$ motif positioned closest to the C-terminal side and the C-terminal of the domain sequence. For example, in the case of the domain sequence illustrated in Fig. 1, w is $4 + 50 + 4 + 100 + 4 + 10 + 4 + 20 + 4 + 30 = 230$ (the $(A)_n$ motif positioned closest to the C-terminal side is excluded.). Next, z/w (%) can be calculated by dividing z by w.

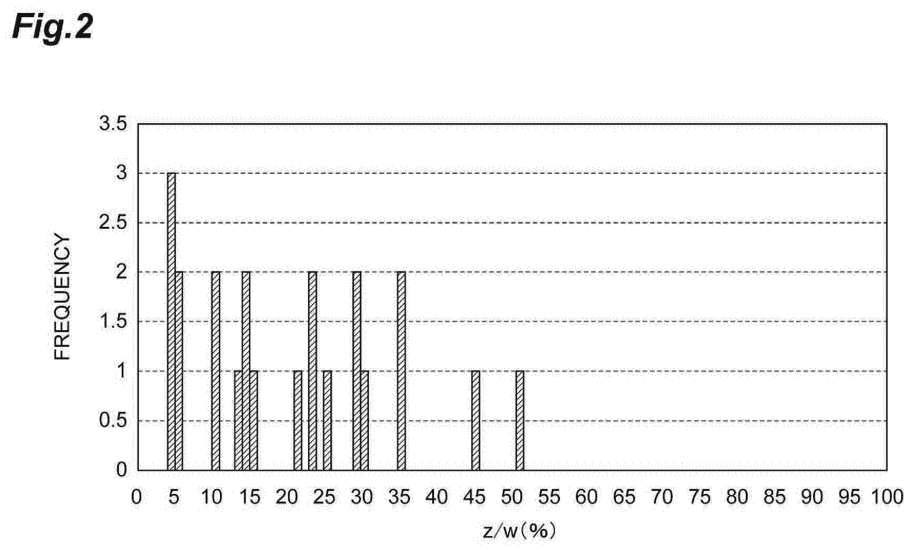

Here, z/w in a naturally occurring fibroin will be described. First, fibroins with amino acid sequence information registered in NCBI GenBank was checked by the exemplified method as described above, and 663 types of fibroins (of which 415 types were fibroins derived from spiders) were extracted. Among all the extracted fibroins, z/w was calculated from an amino acid sequence of a naturally occurring fibroin that contains a domain sequence represented by Formula 1: $[(A)_n$ motif-REP$]_m$, and that has a content ratio of the amino acid sequence consisting of GGX in the fibroin of 6% or less, using the above-mentioned calculation method. The results are shown in Fig. 2. In Fig. 2, the horizontal axis represents z/w (%), and the vertical axis represents frequency. As apparent from Fig. 2, z/w in all the naturally occurring fibroin is less than 50.9% (highest value: 50.86%).

In the second modified fibroin, z/w is preferably 50.9% or more, more preferably 56.1% or more, still more preferably 58.7% or more, even still more preferably 70% or more, and further still preferably 80% or more. The upper limit of z/w is not particularly limited, but may be, for example, 95% or less.

The second modified fibroin may be obtained, for example, by modifying a cloned gene sequence of a naturally occurring fibroin such that at least a part of the nucleotide sequence encoding a glycine residue is substituted to encode another amino acid residue. In this case, one glycine residue in the GGX motif and the GPGXX motif may be selected as the glycine residue to be modified, and also, substitution may be performed so that z/w becomes 50.9% or more. In addition, the second modified fibroin may also be obtained, for example, by designing an amino acid sequence that satisfies the above embodiment based on the amino acid sequence of a naturally occurring fibroin, and chemically synthesizing a nucleic acid encoding the designed amino acid sequence. In any case, in addition to the modification that corresponds to substitution of the glycine residue in REP in the amino acid sequence of a naturally occurring fibroin with another amino acid residue, a modification of the amino acid sequence that corresponds to substitution, deletion, insertion, and/or addition of one or a plurality of amino acid residues may further be performed.

The above another amino acid residue is not particularly limited as long as it is an amino acid residue other than the glycine residue, but is preferably a hydrophobic amino acid residue such as a valine (V) residue, a leucine (L) residue, a isoleucine (I) residue, a methionine (M) residue, a proline (P) residue, a phenylalanine (F) residue, and a tryptophan (W) residue, or a hydrophilic amino acid residues such a glutamine (Q) residue, an asparagine (N) residue, a serine (S) residue, a lysine (K) residue, and a glutamic acid (E) residue, more preferably the valine (V) residue, the leucine (L) residue, the isoleucine (I) residue, the phenylalanine (F) residue, and the glutamine (Q) residue, and still more preferably the glutamine (Q) residue.

More specific examples of the second modified fibroin include a modified fibroin containing (2-i) the amino acid sequence set forth in SEQ ID NO: 6 (Met-PRT380), SEQ ID NO: 7 (Met-PRT410), SEQ ID NO: 8 (Met-PRT525), or SEQ ID NO: 9 (Met-PRT799), or (2-ii) an amino acid sequence having 90% or more sequence identity with the amino acid sequence set forth in SEQ ID NO: 6, SEQ ID NO: 7, SEQ ID NO: 8, or SEQ ID NO: 9.

The modified fibroin (2-i) will be described. The amino acid sequence set forth in SEQ ID NO: 6 is an amino acid sequence in which all GGXs in REP of the amino acid sequence set forth in SEQ ID NO: 10 (Met-PRT313) corresponding to a naturally occurring fibroin are substituted with GQXs. The amino acid sequence set forth in SEQ ID NO: 7 is an amino acid sequence in which every third $(A)_n$ motif from the N-terminal side to the C-terminal side is deleted from the amino acid sequence set forth in SEQ ID NO: 6, and further, one $[(A)_n$ motif-REP$]$ is inserted before the C-terminal sequence. The amino acid sequence set forth in SEQ ID NO: 8 is an amino acid sequence in which two alanine residues are inserted at the C-terminal side of each $(A)_n$ motif of the amino acid sequence set forth in SEQ ID NO: 7, and further some of the glutamine (Q) residues are substituted with a serine (S) residue, and some of the amino acids on the C-terminal side are deleted so that the molecular weight is almost the same as that of SEQ ID NO 7. The amino acid sequence set forth in SEQ ID NO: 9 is an amino acid sequence in which a predetermined hinge sequence and a His tag sequence are added to the C-terminal of a sequence having four-time repetition of a region of 20 domain sequences present in the amino acid sequence set forth in SEQ ID NO: 7 (however, with several amino acid residues on the C-terminal side of the region are substituted).

The value of z/w in the amino acid sequence set forth SEQ ID NO: 10 (corresponding to a naturally occurring fibroin) is 46.8%. The values of z/w in the amino acid sequence set forth in SEQ ID NO: 6, the amino acid sequence set forth in SEQ ID NO: 7, the amino acid sequence set forth in SEQ ID NO: 8, and the amino acid sequence set forth in SEQ ID NO: 9 are 58.7%, 70.1%, 66.1%, and 70.0%, respectively. In addition, the values of x/y at the Giza ratio (which will be described later) of 1:1.8 to 11.3 in the amino acid sequences set forth in SEQ ID NO: 10, SEQ ID NO: 6, SEQ ID NO: 7, SEQ ID NO: 8, and SEQ ID NO: 9 are 15.0%, 15.0%, 93.4%, 92.7%, and 89.8%, respectively.

The modified fibroin (2-i) may be a modified fibroin consisting of the amino acid sequence set forth in SEQ ID NO: 6, SEQ ID NO: 7, SEQ ID NO: 8, or SEQ ID NO: 9.

The modified fibroin (2-ii) is a modified fibroin containing an amino acid sequence having 90% or more sequence identity with the amino acid sequence set forth in SEQ ID NO: 6, SEQ ID NO: 7, SEQ ID NO: 8, or SEQ ID NO: 9. The modified fibroin (2-ii) also is a protein containing a domain sequence represented by Formula 1: $[(A)_n$ motif-REP$]_m$. The sequence identity is preferably 95% or more.

The modified fibroin (2-ii) preferably has 90% or more sequence identity with the amino acid sequence set forth in SEQ ID NO: 6, SEQ ID NO: 7, SEQ ID NO: 8, or SEQ ID NO: 9, and that z/w is 50.9% or more, where z is the total number of amino acid residues in the amino acid sequence consisting of XGX (where X represents an amino acid residue other than glycine) contained in REP; and where w is the total number of amino acid residues in REP in the domain sequence.

The second modified fibroin may contain a tag sequence at either or both of the N-terminal and C-terminal. This makes it possible to isolate, immobilize, detect, and visualize the modified fibroin.

The tag sequence may be, for example, an affinity tag utilizing specific affinity (binding property, affinity) with other molecules. Specific examples of the affinity tag include a histidine tag (His tag). The His tag is a short peptide in which about 4 to 10 histidine residues are arranged and has a property of specifically binding to a metal ion such as nickel, and thus, it can be used for isolation of a modified fibroin by a chelating metal chromatography. Specific examples of the tag sequence include an amino acid sequence set forth in SEQ ID NO: 11 (amino acid sequence containing a His tag sequence and a hinge sequence).

In addition, a tag sequence such as a glutathione-S-transferase (GST) that specifically binds to glutathione and a maltose binding protein (MBP) that specifically binds to maltose may also be used.

Furthermore, an "epitope tag" utilizing an antigen-antibody reaction may also be used. By adding a peptide (an epitope) showing antigenicity as a tag sequence, an antibody against the epitope can be bound. Examples of the epitope tag include an HA (peptide sequence of hemagglutinin of an influenza virus) tag, a myc tag, and a FLAG tag. The modified fibroin can be easily purified with high specificity by utilizing the epitope tag.

It is also possible to further use a tag sequence which can be cleaved with a specific protease. By treating a protein adsorbed via the tag sequence with a protease, it is also possible to recover a modified fibroin cleaved from the tag sequence.

More specific examples of the modified fibroin containing the tag sequence include a modified fibroin containing (2-iii) the amino acid sequence set forth in SEQ ID NO: 12 (PRT380), SEQ ID NO: 13 (PRT410), SEQ ID NO: 14 (PRT525), or SEQ ID NO: 15 (PRT799), or (2-iv) an amino acid sequence having 90% or more sequence identity with the amino acid sequence set forth in SEQ ID NO: 12, SEQ ID NO: 13, SEQ ID NO: 14, or SEQ ID NO: 15.

The amino acid sequences set forth in SEQ ID NO: 16 (PRT313), SEQ ID NO: 12, SEQ ID NO: 13, SEQ ID NO: 14, and SEQ ID NO: 15 are amino acid sequences in which the amino acid sequence (containing a His tag sequence and a hinge sequence) set forth in SEQ ID NO: 11 is added to the N-terminal of the amino acid sequences set forth in SEQ ID NO: 10, SEQ ID NO: 6, SEQ ID NO: 7, SEQ ID NO: 8, and SEQ ID NO: 9, respectively.

The modified fibroin (2-iii) may be a modified fibroin consisting of the amino acid sequence set forth in SEQ ID NO: 12, SEQ ID NO: 13, SEQ ID NO: 14, or SEQ ID NO: 15.

The modified fibroin (2-iv) is a modified fibroin containing an amino acid sequence having 90% or more sequence identity with the amino acid sequence set forth in SEQ ID NO: 12, SEQ ID NO: 13, SEQ ID NO: 14, or SEQ ID NO: 15. The modified fibroin (2-iv) also is a protein containing a domain sequence represented by Formula 1: $[(A)_n$ motif-REP$]_m$. The sequence identity is preferably 95% or more.

It is preferable that the modified fibroin (2-iv) has 90% or more sequence identity with the amino acid sequence set forth in SEQ ID NO: 12, SEQ ID NO: 13, SEQ ID NO: 14, or SEQ ID NO: 15, and that z/w is 50.9% or more, where z is the total number of amino acid residues of the amino acid sequence consisting of XGX (where X represents an amino acid residue other than glycine) contained in REP; and where w is the total number of amino acid residues in REP in the domain sequence.

The second modified fibroin may contain a secretory signal for releasing the protein produced in a recombinant protein production system to the outside of the host. The sequence of the secretory signal may be set as appropriate, depending on the type of the host.

The third modified fibroin has a domain sequence that has an amino acid sequence with a reduced content of $(A)_n$ motifs, as compared with a naturally occurring fibroin. It can be said that the domain sequence of the third modified fibroin is a domain sequence having an amino acid sequence that corresponds to an amino acid sequence in which at least one or a plurality of the $(A)_n$ motifs are deleted, as compared with a naturally occurring fibroin.

The third modified fibroin may be a modified fibroin having an amino acid sequence that corresponds to an amino acid sequence in which 10% to 40% of the $(A)_n$ motifs are deleted from a naturally occurring fibroin.

The domain sequence of the third modified fibroin may be a domain sequence having an amino acid sequence that corresponds to an amino acid sequence in which at least one $(A)_n$ motif for every one to three $(A)_n$ motifs from the N-terminal side to the C-terminal side is deleted, as compared with a naturally occurring fibroin.

The domain sequence of the third modified fibroin may be a domain sequence having an amino acid sequence that corresponds to an amino acid sequence in which at least deletion of two consecutive $(A)_n$ motifs and deletion of one $(A)_n$ motif are repeated in this order from the N-terminal side to the C-terminal side, as compared with a naturally occurring fibroin.

The domain sequence of the third modified fibroin may be a domain sequence having an amino acid sequence that corresponds to an amino acid sequence in which, at least, every third $(A)_n$ motif from the N-terminal side to the C-terminal side is deleted.

The third modified fibroin may be a modified fibroin that contains a domain sequence represented by Formula 1: $[(A)_n \text{ motif-REP}]_m$, and that has an amino acid sequence in which, when the numbers of amino acid residues of REP of two adjacent $[(A)_n \text{ motif-REP}]$ units are sequentially compared from the N-terminal side to the C-terminal side, x/y is 20% or more, 30% or more, 40% or more, or 50% or more, where x is a maximum value of a total value obtained by adding the numbers of amino acid residues of two adjacent $[(A)_n \text{ motif- REP}]$ units wherein when the number of amino acid residues of one REP having less amino acid residues is taken as 1, the ratio of the number of amino acids residues of the other REP satisfies 1.8 to 11.3; and where y is a total number of amino acid residues of the domain sequence. The number of alanine residues with respect to a total number of amino acid residues in the $(A)_n$ motif may be 83% or more, but is preferably 86% or more, more preferably 90% or more, still more preferably 95% or more, and even still more preferably 100% (which means that the $(A)_n$ motif consists only of alanine residues).

A method for calculating x/y will be described in more detail with reference to Fig. 1. Fig. 1 illustrates a domain sequence obtained by removing an N-terminal sequence and a C-terminal sequence from a modified fibroin. The domain sequence has a sequence of, from the N-terminal side (left side), $(A)_n$ motif-the first REP (50 amino acid residues)-$(A)_n$ motif-the second REP (100 amino acid residues)-$(A)_n$ motif-the third REP (10 amino acid residues)-$(A)_n$ motif-the fourth REP (20 amino acid residues)-$(A)_n$ motif-the fifth REP (30 amino acid residues)-$(A)_n$ motif.

The two adjacent $[(A)_n \text{ motif-REP}]$ units are sequentially selected from the N-terminal side to the C-terminal side so that there is no overlap. There may be $[(A)_n \text{ motif-REP}]$ unit that is not selected. Fig. 1 illustrates Pattern 1 (a comparison of the first REP and the second REP, and a comparison of the third REP and the fourth REP), Pattern 2 (a comparison of the first REP and the second REP, and a comparison of the fourth REP and the fifth REP), Pattern 3 (a comparison of the second REP and the third REP, and a comparison of the fourth REP and the fifth REP), and Pattern 4 (a comparison of the first REP and the second REP). There are selection methods other than these.

Next, for each pattern, the number of amino acid residues of each REP in the selected adjacent two $[(A)_n \text{ motif-REP}]$ units is compared. The comparison is performed by determining the ratio, relative to one REP having less amino acid residues taken as 1, of the number of amino acid residues of the other REP. For example, in a case of comparing the first REP (50 amino acid residues) and the second REP (100 amino acid residues), the ratio of the number of amino acid residues of the second REP is 100/50 = 2, where the first REP having less amino acid residues is taken as 1. Similarly, in a case of comparing the fourth REP (20 amino acid residues) and the fifth REP (30 amino acid residues), the ratio of the number of amino acid residues of the fifth REP is 30/20 = 1.5, where the fourth REP having less amino acid residues is taken as 1.

In Fig. 1, a set of $[(A)_n \text{ motif-REP}]$ units in which, where one REP having less amino acid residues is taken as 1, the ratio of the number of amino acid residues of the other REP is 1.8 to 11.3, is indicated by a solid line. In the present specification, this ratio is referred to as a Giza ratio. A set of $[(A)_n \text{ motif-REP}]$ units in which, where a REP having less amino acid residues is taken as 1, the ratio of the number of amino acid residues of the other REP is less than 1.8 or more than 11.3, is indicated by a dotted line.

In each pattern, the numbers of all amino acid residues (not only REP but also the number of amino acid residues in the $(A)_n$ motif) of the two adjacent $[(A)_n \text{ motif-REP}]$ units indicated by a solid line are added. Then, the total values obtained by addition are compared, and the total value of the pattern having the highest total value (a maximum value of the total value) is defined as x. In the example illustrated in Fig. 1, the total value of Pattern 1 is the maximum.

Next, x/y (%) can be calculated by dividing x by the total number y of the amino acid residues of the domain sequence.

In the third modified fibroin, x/y is preferably 50% or more, more preferably 60% or more, still more preferably 65% or more, even still more preferably 70% or more, further still preferably 75% or more, and particularly preferably 80% or more. The upper limit of x/y is not particularly limited, but may be, for example, 100% or less. In a case where the Giza ratio is 1: 1.9 to 11.3, x/y is preferably 89.6% or more, in a case where the Giza ratio is 1:1.8 to 3.4, x/y is more preferably 77.1% or more, in a case where the Giza ratio is 1: 1.9 to 8.4, x/y is still more preferably 75.9% or more, and in a case where the Giza ratio is 1:1.9 to 4.1, x/y is even still more preferably 64.2% or more.

In a case where the third modified fibroin is a modified fibroin in which at least seven of a plurality of $(A)_n$ motifs present in the domain sequence are composed only of alanine residues, x/y is preferably 46.4% or more, more preferably 50% or more, still more preferably 55% or more, even still more preferably 60% or more, further still preferably 70% or more, and particularly preferably 80% or more. The upper limit of x/y is not particularly limited and it only needs to be 100% or less.

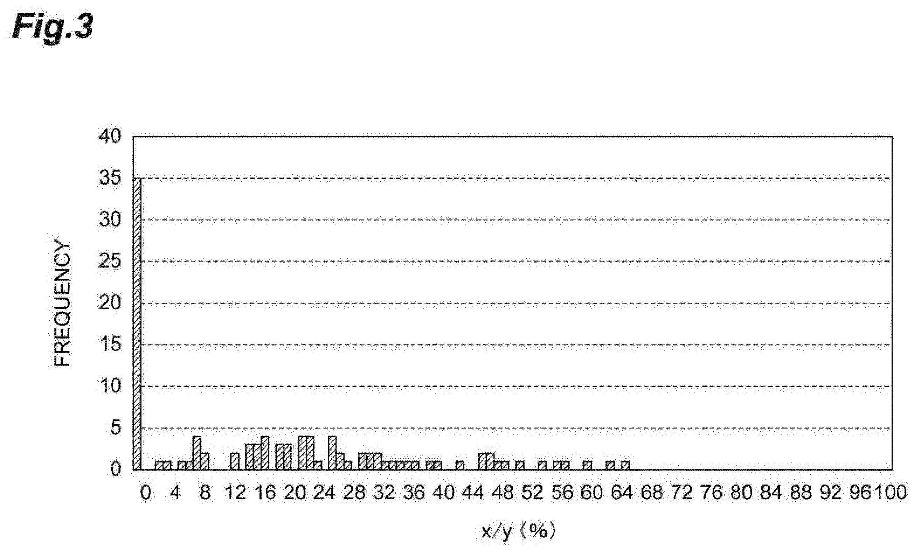

Here, x/y in a naturally occurring fibroin will be described. First, fibroins with amino acid sequence information registered in NCBI GenBank was checked by the exemplified method as described above, and 663 types of fibroins (of which 415 types were fibroins derived from spiders) were extracted. Among all the extracted fibroins, x/y was calculated from an amino acid sequence of a naturally occurring fibroin constituted with a domain sequence represented by Formula 1: $[(A)_n$ motif-REP$]_m$, using the above-mentioned calculation method. The results in a case where the Giza ratio is 1:1.9 to 4.1 are shown in Fig. 3.

In Fig. 3, the horizontal axis represents x/y (%) and the vertical axis represents a frequency. As apparent from Fig. 3, x/y in all the naturally occurring fibroin is less than 64.2% (highest value: 64.14%).

The third modified fibroin may be obtained, for example, by deleting one or a plurality sequences encoding $(A)_n$ motif from a cloned gene sequence of a naturally occurring fibroin so that x/y is 64.2% or more. In addition, the third modified fibroin may also be obtained, for example, by designing an amino acid sequence that corresponds to an amino acid sequence in which one or a plurality $(A)_n$ motifs are deleted from an amino acid sequence of a naturally occurring fibroin so that x/y becomes 64.2% or more, and chemically synthesizing a nucleic acid encoding the designed amino acid sequence. In any case, in addition to the modification that corresponds to deletion of the $(A)_n$ motif from an amino acid sequence of a naturally occurring fibroin, a modification of the amino acid sequence that corresponds to substitution, deletion, insertion, and/or addition of one or a plurality of amino acid residues may further be performed.

More specific examples of the third modified fibroin include a modified fibroin containing an amino acid sequence having (3-i) the amino acid sequence set forth in SEQ ID NO: 17 (Met-PRT399), SEQ ID NO: 7 (Met-PRT410), SEQ ID NO: 8 (Met-PRT525), or SEQ ID NO: 9 (Met-PRT799), or (3-ii) an amino acid sequence having 90% or more sequence identity with the amino acid sequence set forth in SEQ ID NO: 17, SEQ ID NO: 7, SEQ ID NO: 8, or SEQ ID NO: 9.

The modified fibroin (3-i) will be described. The amino acid sequence set forth in SEQ ID NO: 17 is an amino acid sequence in which every third $(A)_n$ motif from the N-terminal side to the C-terminal side is deleted from the amino acid sequence set forth in SEQ ID NO: 10 (Met-PRT313) corresponding to a naturally occurring fibroin, and further, one $[(A)_n$ motif-REP] is inserted before the C-terminal sequence. The amino acid sequences set forth in SEQ ID NO: 7, SEQ ID NO: 8, or SEQ ID NO: 9 are as described for the second modified fibroin.

The value of x/y at a Giza ratio of 1:1.8 to 11.3 in the amino acid sequence set forth in SEQ ID NO: 10 (corresponding to a naturally occurring fibroin) is 15.0%. Both the values of x/y in the amino acid sequence set forth in SEQ ID NO: 17 and the amino acid sequence set forth in SEQ ID NO: 7 are 93.4%. The value of x/y in the amino acid sequence set forth in SEQ ID NO: 8 is 92.7%. The value of x/y in the amino acid sequence set forth in SEQ ID NO: 9 is 89.8%. The values of z/w in the amino acid sequences set forth in SEQ ID NO: 10, SEQ ID NO: 17, SEQ ID NO: 7, SEQ ID NO: 8, and SEQ ID NO: 9 are 46.8%, 56.2%, 70.1%, 66.1%, and 70.0%, respectively.

The modified fibroin (3-i) may be a modified fibroin consisting of the amino acid sequence set forth in SEQ ID NO: 17, SEQ ID NO: 7, SEQ ID NO: 8, or SEQ ID NO: 9.

The modified fibroin (3-ii) is a modified fibroin containing an amino acid sequence having 90% or more sequence identity with the amino acid sequence set forth in SEQ ID NO: 17, SEQ ID NO: 7, SEQ ID NO: 8, or SEQ ID NO: 9. The modified fibroin (3-ii) also is a protein containing a domain sequence represented by Formula 1: $[(A)_n$ motif-REP$]_m$. The sequence identity is preferably 95% or more.

It is preferable that the modified fibroin (3-ii) has 90% or more sequence identity with the amino acid sequence set forth in SEQ ID NO: 17, SEQ ID NO: 7, SEQ ID NO: 8, or SEQ ID NO: 9, and that, when the numbers of amino acid residues of REP of two adjacent $[(A)_n$ motif-REP] units are sequentially compared from the N-terminal side to the C-terminal side, x/y is 64.2% or more, where x is a maximum value of a total value obtained by adding the numbers of amino acid residues of two adjacent $[(A)_n$ motif-REP] units wherein when the number of amino acid residues of one REP having less amino acid residues is taken as 1, the ratio of the number of amino acids residues of the other REP satisfies 1.8 to 11.3 (the Giza ratio is 1:1.8 to 11.3); and where y is a total number of amino acid residues of the domain sequence.

The third modified fibroin may contain a tag sequence described above at either or both of the N-terminal and C-terminal.

A more specific example of the modified fibroin containing a tag sequence may be a modified fibroin containing (3-iii) the amino acid sequence set forth in SEQ ID NO: 18 (PRT399), SEQ ID NO: 13 (PRT410), SEQ ID NO: 14 (PRT525), or SEQ ID NO: 15 (PRT799), or (3-iv) an amino acid sequence having 90% or more sequence identity with the amino acid sequence set forth in SEQ ID NO: 18, SEQ ID NO: 13, SEQ ID NO: 14, or SEQ ID NO: 15.

The amino acid sequences set forth in SEQ ID NO: 18, SEQ ID NO: 13, SEQ ID NO: 14, and SEQ ID NO: 15 are amino acid sequences in which the amino acid sequence (containing a His tag sequence and a hinge sequence) set forth in SEQ ID NO: 11 is added to the N-terminal of the amino acid sequences set forth in SEQ ID NO: 17, SEQ ID NO: 7, SEQ ID NO: 8, and SEQ ID NO: 9, respectively.

The modified fibroin (3-iii) may be a modified fibroin consisting of the amino acid sequence set forth in SEQ ID NO: 18, SEQ ID NO: 13, SEQ ID NO: 14, or SEQ ID NO: 15.

The modified fibroin (3-iv) is a modified fibroin containing an amino acid sequence having 90% or more sequence identity with the amino acid sequence set forth in SEQ ID NO: 18, SEQ ID NO: 13, SEQ ID NO: 14, or SEQ ID NO: 15. The modified fibroin (3-iv) also is a protein containing a domain sequence represented by Formula 1: $[(A)_n \text{ motif-REP}]_m$. The sequence identity is preferably 95% or more.

It is preferable that the modified fibroin (3-iv) has 90% or more sequence identity with the amino acid sequence set forth in SEQ ID NO: 18, SEQ ID NO: 13, SEQ ID NO: 14 or SEQ ID NO: 15, and that, when the numbers of amino acid residues of REP of two adjacent $[(A)_n \text{ motif-REP}]$ units are sequentially compared from the N-terminal side to the C-terminal side, x/y is 64.2% or more, where x is a maximum value of a total value obtained by adding the numbers of amino acid residues of two adjacent $[(A)_n \text{ motif-REP}]$ units wherein when the number of amino acid residues of one REP having less amino acid residues is taken as 1, the ratio of the number of amino acids residues of the other REP satisfies 1.8 to 11.3; and where y is a total number of amino acid residues of the domain sequence.

The third modified fibroin may contain a secretory signal for releasing the protein produced in a recombinant protein production system to the outside of the host. The sequence of the secretory signal may be set as appropriate, depending on the type of the host.

The fourth modified fibroin has a domain sequence that has an amino acid sequence with a reduced content of glycine residues, in addition to the reduced content of $(A)_n$ motifs, as compared with a naturally occurring fibroin. It can be said that the domain sequence of the fourth modified fibroin is a domain sequence further having an amino acid sequence that corresponds to an amino acid sequence in which at least one or a plurality of glycine residues in REP are substituted with other amino acid residues, in addition to the amino acid sequence in which at least one or a plurality of the $(A)_n$ motifs are deleted, as compared with a naturally occurring fibroin. That is, the fourth modified fibroin is a modified fibroin having both the characteristics of the second modified fibroin and the characteristics of the third modified fibroin described above. Specific embodiments and the like are as described for the second modified fibroin and the third modified fibroin.

More specific examples of the fourth modified fibroin include a modified fibroin containing (4-i) the amino acid sequence set forth in SEQ ID NO: 7 (Met-PRT410), SEQ ID NO: 8 (Met-PRT525), SEQ ID NO: 9 (Met-PRT799), SEQ ID NO: 13 (PRT410), SEQ ID NO: 14 (PRT525), or SEQ ID NO: 15 (PRT799), or (4-ii) an amino acid sequence having 90% or more sequence identity with the amino acid sequence set forth in SEQ ID NO: 7, SEQ ID NO: 8, SEQ ID NO: 9, SEQ ID NO: 13, SEQ ID NO: 14, or SEQ ID NO: 15. Specific embodiments of the modified fibroin containing the amino acid sequence set forth in SEQ ID NO: 7, SEQ ID NO: 8, SEQ ID NO: 9, SEQ ID NO: 13, SEQ ID NO: 14, or SEQ ID NO: 15 are as described above.

The fifth modified fibroin may have a domain sequence that has an amino acid sequence containing a region having a locally high hydropathy index, which corresponds to an amino acid sequence in which one or a plurality of amino acid residues in REP are substituted with an amino acid residue having a high hydropathy index and/or one or a plurality of amino acid residues having a high hydropathy index is inserted into REP, as compared with a naturally occurring fibroin.

It is preferable that the region having a locally high hydropathy index is composed of 2 to 4 consecutive amino acid residues.

It is more preferable that the amino acid residues having a high hydropathy index are selected from isoleucine (I), valine (V), leucine (L), phenylalanine (F), cysteine (C), methionine (M), and alanine (A).

The fifth modified fibroin may further have a modification of an amino acid sequence that corresponds to substitution, deletion, insertion, and/or addition of one or a plurality of amino acid residues, as compared with a naturally occurring fibroin, in addition to the modification that corresponds to substitution of one or a plurality of amino acid residues in REP with amino acid residues having a high hydropathy index and/or insertion of one or a plurality of amino acid residues having a high hydropathy index into REP, as compared with a naturally occurring fibroin.

The fifth modified fibroin may be obtained, for example, from a cloned gene sequence of a naturally occurring fibroin, by substituting one or a plurality of hydrophilic amino acid residues in REP (for example, amino acid residues having a negative hydropathy index) with a hydrophobic amino acid residue (for example, amino acid residues having a positive hydropathy index), and/or inserting one or a plurality of hydrophobic amino acid residues into REP. In addition, the fifth modified fibroin may also be obtained, for example, by designing an amino acid sequence that corresponds to an amino acid sequence in which one or a plurality of hydrophilic amino acid residues in REP are substituted with hydrophobic amino acid residues and/or one or a plurality of hydrophobic amino acid residues are inserted into REP, from an amino acid sequence of a naturally occurring fibroin, and chemically synthesizing a nucleic acid encoding the designed amino acid sequence. In any case, in addition to the modification that corresponds to substitution of one or a plurality of hydrophilic amino acid residues in REP with hydrophobic amino acid residues and/or insertion of one or a plurality of hydrophobic amino acid residues into REP in an amino acid sequence of a naturally occurring fibroin, a modification of an amino acid sequence that corresponds to substitution, deletion, insertion, and/or addition of one or a plurality of amino acid residues may further be performed.

The fifth modified fibroin may be a modified fibroin that contains a domain sequence represented by Formula 1: $[(A)_n \text{ motif-REP}]_m$, and that has an amino acid sequence in which p/q is 6.2% or more, where p is the total number of amino acid residues contained in a region where an average value of hydropathy indices of the four consecutive amino acid residues is 2.6 or more in all REPs contained in the domain sequence excluding the sequence between the $(A)_n$ motif positioned closest to the C-terminal side and the C-terminal of the domain sequence; and where q is the total number of amino acid residues contained in the domain sequence excluding the sequence between the $(A)_n$ motif positioned closest to the C-terminal side and the C-terminal of the domain sequence.

With regard to the hydropathy index of an amino acid residue, known index from (Hydropathy index: Kyte J, & Doolittle R (1982) "A simple method for displaying the hydropathic character of a protein", J. Mol. Biol., 157, pp. 105-132) may be used. Specifically, the hydropathy index (hereinafter also referred to as "HI") of each amino acid is as shown in Table 1 below.

[Table 1]

| Amino acid | HI | Amino acid | HI |
|---|---|---|---|
| Isoleucine (Ile) | 4.5 | Tryptophan (Trp) | -0.9 |
| Valine (Val) | 4.2 | Tyrosine (Tyr) | -1.3 |
| Leucine (Leu) | 3.8 | Proline (Pro) | -1.6 |
| Phenylalanine (Phe) | 2.8 | Histidine (His) | -3.2 |
| Cysteine (Cys) | 2.5 | Asparagine (Asn) | -3.5 |
| Methionine (Met) | 1.9 | Aspartic acid (Asp) | -3.5 |
| Alanine (Ala) | 1.8 | Glutamine (Gln) | -3.5 |
| Glycine (Gly) | -0.4 | Glutamic acid (Glu) | -3.5 |
| Threonine (Thr) | -0.7 | Lysine (Lys) | -3.9 |
| Serine (Ser) | -0.8 | Arginine (Arg) | -4.5 |

A method for calculating p/q will be described in more detail. For calculation, a sequence (hereinafter also referred to as a "Sequence A") obtained by removing, from domain sequence represented by Formula 1: $[(A)_n \text{ motif-REP}]_m$, the sequence between the $(A)_n$ motif positioned closest to the C-terminal side and the C-terminal of the domain sequence is used. First, in all REPs contained in Sequence A, average values of hydropathy indices of the four consecutive amino acid residues are calculated. The average value of the hydropathy indices is obtained by dividing the sum of HI of each of the amino acid residues contained in the four consecutive amino acid residues by 4 (the number of amino acid residues). The average value of the hydropathy indices is obtained for all four consecutive amino acid residues (each of the amino acid residues is used for calculating the average value 1 to 4 times). Next, a region where the average value of the hydropathy indices of the four consecutive amino acid residues is 2.6 or more is identified. Even if a certain amino acid residues correspond to a plurality of the "four consecutive amino acid residues having an average value of the hydropathy indices of 2.6 or more", the amino acid residue is counted as one amino acid residue in the region. The total number of amino acid residues contained in the region is p. Further, the total number of amino acid residues contained in Sequence A is q.

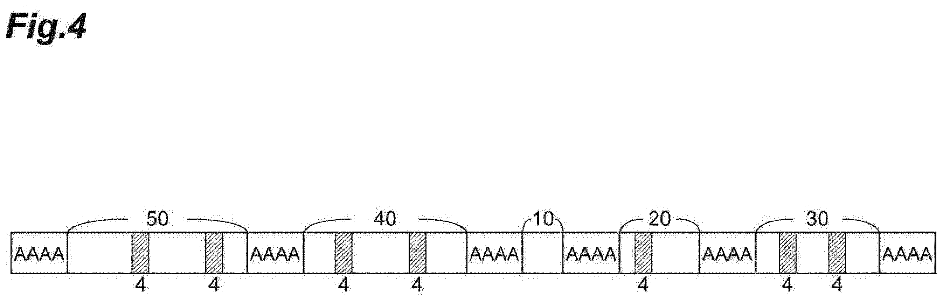

For example, in a case where 20 "four consecutive amino acid residues having an average value of the hydropathy indices of 2.6 or more " are extracted (without an overlap), there are 20 of the four consecutive amino acid residues (without an overlap) in the region in which the average value of the hydropathy indices of the four consecutive amino acid residues is 2.6 or more, and thus p is $20 \times 4 = 80$. In addition, for example, in a case where two "four consecutive amino acid residues having an average value of the hydropathy indices of 2.6 or more" overlap by only one amino acid residue, seven amino acid residues are contained in the region in which the average value of the hydropathy indices of the four consecutive amino acid residues is 2.6 or more ($p = 2 \times 4 - 1 = 7$; "-1" is deduction for overlap). For example, in a case of the domain sequence shown in Fig. 4, since there are seven "four consecutive amino acid residues having an average value of the hydropathy indices of 2.6 or more" without an overlap, p is $7 \times 4 = 28$. Further, for example, in a case of the domain sequence illustrated in Fig. 4, q is $4 + 50 + 4 + 40 + 4 + 10 + 4 + 20 + 4 + 30 = 170$ (the $(A)_n$ motif present at the end of the C-terminal side is not included). Next, p/q (%) can be calculated by dividing p by q. In the case of Fig. 4, p/q (%) is 28/170 = 16.47%.

In the fifth modified fibroin, p/q is preferably 6.2% or more, more preferably 7% or more, still more preferably 10% or more, even still more preferably 20% or more, and further still preferably 30% or more. The upper limit of p/q is not particularly limited, but may be, for example, 45% or less.

The fifth modified fibroin may be obtained, for example, by modifying an amino acid sequence of a cloned a naturally occurring fibroin to an amino acid sequence containing a region having a locally high hydropathy index, by substituting one or a plurality of hydrophilic amino acid residues in REP (for example, amino acid residues having a negative hydropathy index) with hydrophobic amino acid residues (for example, amino acid residues having a positive hydropathy index), and/or inserting one or a plurality of hydrophobic amino acid residues into REP, so as to satisfy the above condition of p/q. In addition, the modified fibroin may also be obtained, for example, by designing an amino acid sequence satisfying the above-described condition of p/q based on the amino acid sequence of a naturally occurring fibroin, and chemically synthesizing a nucleic acid encoding the designed amino acid sequence. In any case, in addition to the modification that corresponds substitution of one or a plurality of amino acid residues in REP with amino acid residues having a high hydropathy index and/or insertion of one or a plurality of amino acid residues having a high hydropathy index, as compared with a naturally occurring fibroin, a modification that corresponds to substitution, deletion, insertion, and/or addition of one or a plurality of amino acid residues may further be performed.

The amino acid residue having a high hydropathy index is not particularly limited, but is preferably isoleucine (I), valine (V), leucine (L), phenylalanine (F), cysteine (C), methionine (M), and alanine (A), and more preferably valine (V), leucine (L), and isoleucine (I).

More specific examples of the fifth modified fibroin contains a modified fibroin containing an amino acid sequence having (5-i) the amino acid sequence set forth in SEQ ID NO: 19 (Met-PRT720), SEQ ID NO: 20 (Met-PRT665), or SEQ ID NO: 21 (Met-PRT666), or (5-ii) an amino acid sequence having 90% or more sequence identity with the amino acid sequence set forth in SEQ ID NO: 19, SEQ ID NO: 20, or SEQ ID NO: 21.

The modified fibroin (5-i) will be described. The amino acid sequence set forth in SEQ ID NO: 19 is an amino acid sequence in which two amino acid sequences, each consisting of three amino acid residues (VLI), are inserted for every other REP excluding the terminal domain sequence on the C-terminal side; further, some of the glutamine (Q) residues are substituted with serine (S) residues; and some of the amino acids on the C-terminal side are deleted, with respect to the amino acid sequence set forth in SEQ ID NO: 7 (Met-PRT410). The amino acid sequence set forth in SEQ ID NO: 20 is an amino acid sequence in which one amino acid sequence consisting of three amino acid residues (VLI) is inserted for every other REP with respect to the amino acid sequence set forth in SEQ ID NO: 8 (Met-PRT525). The amino acid sequence set forth in SEQ ID NO: 21 is an amino acid sequence in which two amino acid sequences each consisting of three amino acid residues (VLI) are inserted for every other REP with respect to the amino acid sequence set forth in SEQ ID NO: 8.

The modified fibroin (5-i) may be a modified fibroin consisting of the amino acid sequence set forth in SEQ ID NO: 19, SEQ ID NO: 20, or SEQ ID NO: 21.

The modified fibroin (5-ii) is a modified fibroin containing an amino acid sequence having 90% or more sequence identity with the amino acid sequence set forth in SEQ ID NO: 19, SEQ ID NO: 20, or SEQ ID NO: 21. The modified fibroin (5-ii) also is a protein containing a domain sequence represented by Formula 1: $[(A)_n$ motif-REP$]_m$. The sequence identity is preferably 95% or more.

It is preferable that the modified fibroin (5-ii) has 90% or more sequence identity with the amino acid sequence set forth in SEQ ID NO: 19, SEQ ID NO: 20, or SEQ ID NO: 21, and that p/q is 6.2% or more, where p is the total number of amino acid residues contained in a region where an average value of hydropathy indices of the four consecutive amino acid residues is 2.6 or more in all REPs contained in the domain sequence excluding the sequence between the $(A)_n$ motif positioned closest to the C-terminal side and the C-terminal of the domain sequence; and where q is the total number of amino acid residues contained in the domain sequence excluding the sequence between the $(A)_n$ motif positioned closest to the C-terminal side and the C-terminal of the domain sequence.

The fifth modified fibroin may contain a tag sequence at either or both of the N-terminal and C-terminal.

More specific examples of the modified fibroin containing a tag sequence include a modified fibroin containing (5-iii) the amino acid sequence set forth in SEQ ID NO: 22 (PRT720), SEQ ID NO: 23 (PRT665), or SEQ ID NO: 24 (PRT666), or (5-iv) an amino acid sequence having 90% or more sequence identity with the amino acid sequence set forth in SEQ ID NO: 22, SEQ ID NO: 23, or SEQ ID NO: 24.

The amino acid sequences set forth in SEQ ID NO: 22, SEQ ID NO: 23, and SEQ ID NO: 24 are amino acid sequences in which the amino acid sequence (containing a His tag sequence and a hinge sequence) set forth in SEQ ID NO: 11 is added to the N-terminal of the amino acid sequences set forth in SEQ ID NO: 19, SEQ ID NO: 20, and SEQ ID NO: 21, respectively.

The modified fibroin (5-iii) may be a modified fibroin consisting of the amino acid sequence set forth in SEQ ID NO: 22, SEQ ID NO: 23, or SEQ ID NO: 24.

The modified fibroin (5-iv) is a modified fibroin containing an amino acid sequence having 90% or more sequence identity with the amino acid sequence set forth in SEQ ID NO: 22, SEQ ID NO: 23, or SEQ ID NO: 24. The modified fibroin (5-iv) also is a protein containing a domain sequence represented by Formula 1: $[(A)_n$ motif-REP$]_m$. The sequence identity is preferably 95% or more.

It is preferable that the modified fibroin (5-iv) has 90% or more sequence identity with the amino acid sequence set forth in SEQ ID NO: 22, SEQ ID NO: 23, or SEQ ID NO: 24, and that p/q is 6.2% or more, where p is the total number of amino acid residues contained in a region where an average value of hydropathy indices of the four consecutive amino acid residues is 2.6 or more in all REPs contained in the domain sequence excluding the sequence between the $(A)_n$ motif positioned closest to the C-terminal side and the C-terminal of the domain sequence; and where q is the total number of amino acid residues contained in the domain sequence excluding the sequence between the $(A)_n$ motif positioned closest to the C-terminal side and the C-terminal of the domain sequence.

The fifth modified fibroin may contain a secretory signal for releasing the protein produced in a recombinant protein production system to the outside of the host. The sequence of the secretory signal may be set as appropriate, depending on the type of the host.

The sixth modified fibroin has an amino acid sequence with a reduced content of glutamine residues, as compared with a naturally occurring fibroin.

It is preferable that the sixth modified fibroin contains at least one motif selected from a GGX motif and a GPGXX motif in the amino acid sequence of REP.

In a case where the sixth modified fibroin contains a GPGXX motif in REP, a GPGXX motif content rate is usually 1% or more, may be 5% or more, and is preferably 10% or more. The upper limit of the GPGXX motif content rate is not particularly limited, and it may be 50% or less, and may be 30% or less.

In the present specification, the "GPGXX motif content rate" is a value calculated by the following method.

In a fibroin (a modified fibroin or a naturally occurring fibroin) containing a domain sequence represented by Formula 1: $[(A)_n$ motif-REP$]_m$ or Formula 2: $[(A)_n$ motif-REP$]_m$-$(A)_n$ motif, the GPGXX motif content rate is calculated as s/t, where s is the number obtained by tripling the total number of the GPGXX motifs (namely, corresponds to the total number of G and P in the GPGXX motifs) contained in all REPs contained in the domain sequence excluding the sequence between the $(A)_n$ motif positioned closest to the C-terminal side and the C-terminal of the domain sequence; and where t is the total number of amino acid residues in all REPs obtained by excluding the sequence between the $(A)_n$ motif positioned closest to the C-terminal side and the C-terminal of the domain sequence from the domain sequence and further excluding the $(A)_n$ motifs.

For the calculation of the GPGXX motif content rate, a "domain sequence excluding the sequence between the $(A)_n$ motif positioned closest to the C-terminal side and the C-terminal of the domain sequence" is used, because "the sequence between the $(A)_n$ motif positioned closest to the C-terminal side and the C-terminal of the domain sequence (sequence corresponding to REP) may contain a sequence having a low correlation with the sequence characteristic of a fibroin, which influences the calculation result of the GPGXX motif content rate when m is small (that is, when the domain sequence is short), and thus, this effect is to be eliminated. In a case where a "GPGXX motif" is located at the C-terminal of REP, it is treated as "GPGXX motif" even if "XX" is, for example, "AA".

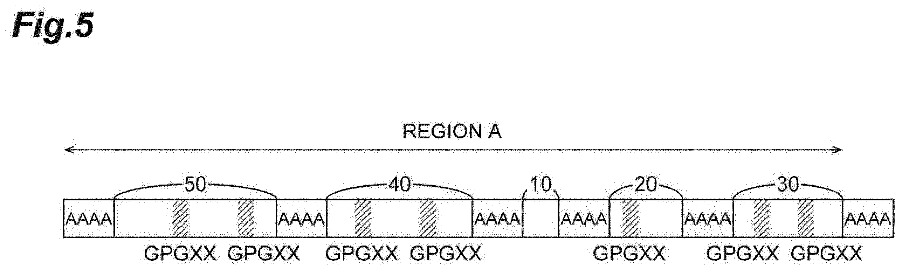

Fig. 5 is a schematic diagram illustrating a domain sequence of a modified fibroin. A method for calculating the GPGXX motif content rate will be specifically described with reference to Fig. 5. First, in a domain sequence of the modified fibroin (which is an $[(A)_n$ motif-REP$]_m$-$(A)_n$ motif] type) illustrated in Fig. 5, the number of GPGXX motifs for calculation of s is 7 and s is $7 \times 3 = 21$, because all REPs are contained in the "domain sequence excluding the sequence between the $(A)_n$ motif positioned closest to the C-terminal side and the C-terminal of the domain sequence" (indicated as "REGION A" in Fig. 5). Similarly, since all REPs are contained in the "domain sequence excluding the sequence between the $(A)_n$ motif positioned closest to the C-terminal side and the C-terminal of the domain sequence" (indicated as "REGION A" in Fig. 5), the total number, t, of amino acid residues in all REPs obtained by further excluding the $(A)_n$ motifs from the "domain sequence excluding the sequence between the $(A)_n$ motif positioned closest to the C-terminal side and the C-terminal of the domain sequence" is $50 + 40 + 10 + 20 + 30 = 150$. Next, s/t (%) can be calculated by dividing s by t, and thus, in a case of the modified fibroin of Fig. 5, s/t (%) is 21/150 = 14.0%.

In the sixth modified fibroin, the glutamine residue content rate is preferably 9% or less, more preferably 7% or less, still more preferably 4% or less, and particularly preferably 0%.

In the present specification, the "glutamine residue content rate" is a value calculated by the following method.

In a fibroin (a modified fibroin or a naturally occurring fibroin) containing a domain sequence represented by Formula 1: $[(A)_n$ motif-REP$]_m$ or Formula 2: $[(A)_n$ motif-REP$]_m$-$(A)_n$ motif, the glutamine residue content rate is calculated as u/t, where u is the total number of glutamine residues contained in all REPs contained in the domain excluding the sequence between the $(A)_n$ motif positioned closest to the C-terminal side and the C-terminal of the domain sequence (sequence corresponding to the "REGION A" in Fig. 5); and where t is the total number of amino acid residues in all REPs obtained by excluding the sequence between the $(A)_n$ motif positioned closest to the C-terminal side and the C-terminal of the domain sequence from the domain sequence and further excluding the $(A)_n$ motifs. The reason as to why the "domain sequence excluding the sequence between the $(A)_n$ motif positioned closest to the C-terminal side and the C-terminal of the domain sequence" is used for the calculation of the glutamine residue content rate is the same as the reason described above.

The domain sequence of the sixth modified fibroin may be a domain sequence having an amino acid sequence that corresponds to an amino acid sequence in which one or a plurality of glutamine residues in REP are deleted or substituted with other amino acid residues, as compared with a naturally occurring fibroin.

The "other amino acid residues" may be amino acid residues other than the glutamine residue, but are preferably amino acid residues having a higher hydropathy index than that of the glutamine residue. The hydropathy indices of the amino acid residues are as shown in Table 1.

As shown in Table 1, examples of the amino acid residues having a higher hydropathy index than a glutamine residue include an amino acid residue selected from isoleucine (I), valine (V), leucine (L), phenylalanine (F), cysteine (C), methionine (M), alanine (A), glycine (G), threonine (T), serine (S), tryptophan (W), tyrosine (Y), proline (P), and histidine (H). Among these, an amino acid residue selected from isoleucine (I), valine (V), leucine (L), phenylalanine (F), cysteine (C), methionine (M), and alanine (A) is more preferable, and an amino acid residue selected from isoleucine (I), valine (V), leucine (L), and phenylalanine (F) is still more preferable.

In the sixth modified fibroin, the degree of hydrophobicity of REP is preferably -0.8 or more, more preferably -0.7 or more, still more preferably 0 or more, even still more preferably 0.3 or more, and particularly preferably 0.4 or more. The upper limit of the degree of hydrophobicity of REP is not particularly limited, and it may be 1.0 or less, and may be 0.7 or less.

In the present specification, the "degree of hydrophobicity of REP" is a value calculated by the following method.

In a fibroin (a modified fibroin or a naturally occurring fibroin) containing a domain sequence represented by Formula 1: $[(A)_n$ motif-REP$]_m$ or Formula 2: $[(A)_n$ motif-REP$]_m$-$(A)_n$ motif, the degree of hydrophobicity of REP is calculated as v/t, where v is the total sum of the hydropathy index of each amino acid residue contained in all REPs contained in the domain a sequence excluding the sequence between the $(A)_n$ motif positioned closest to the C-terminal side and the C-terminal of the domain sequence (sequence corresponding to a "REGION A" in Fig. 5); and where t is the total number of amino acid residues in all REPs obtained by excluding the sequence between the $(A)_n$ motif positioned closest to the C-terminal side and the C-terminal of the domain sequence from the domain sequence and further excluding the $(A)_n$ motifs. The reason as to why the "domain sequence excluding the sequence between the $(A)_n$ motif positioned closest to the C-terminal side and the C-terminal of the domain sequence" is used for the calculation of the degree of hydrophobicity of REP is the same as the reason described above.

The domain sequence of the sixth modified fibroin may further have a modification of an amino acid sequence that corresponds to substitution, deletion, insertion, and/or addition of one or a plurality of amino acid residues, in addition to the modification that corresponds to deletion of one or a plurality of glutamine residues in REP and/or substitution of one or a plurality of glutamine residues in REP with other amino acid residues, as compared with a naturally occurring fibroin.

The sixth modified fibroin may be obtained, for example, from a cloned gene sequence of a naturally occurring fibroin, by deleting one or a plurality of glutamine residues in REP and/or substituting one or a plurality of glutamine residues in REP with other amino acid residues. In addition, the sixth modified fibroin may also be obtained by designing an amino acid sequence that corresponds to an amino acid sequence in which one or a plurality of glutamine residues in REP are deleted, and/or one or a plurality of glutamine residues in REP are substituted with other amino acid residues, based on the amino acid sequence of a naturally occurring fibroin, and chemically synthesizing a nucleic acid encoding the designed amino acid sequence.

More specific examples of the sixth modified fibroin include (6-i) a modified fibroin containing the amino acid sequence set forth in SEQ ID NO: 25 (Met-PRT888), SEQ ID NO: 26 (Met-PRT965), SEQ ID NO: 27 (Met-PRT889), SEQ ID NO: 28 (Met-PRT916), SEQ ID NO: 29 (Met-PRT918), SEQ ID NO: 30 (Met-PRT699), SEQ ID NO: 31 (Met-PRT698), SEQ ID NO: 32 (Met-PRT966), SEQ ID NO: 41 (Met-PRT917), or SEQ ID NO: 42 (Met-PRT1028), or (6-ii) a modified fibroin containing an amino acid sequence having 90% or more sequence identity with the amino acid sequence set forth in SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 41, or SEQ ID NO: 42.

The modified fibroin (6-i) will be described. The amino acid sequence set forth in SEQ ID NO: 25 is obtained by substituting all QQs in an amino acid sequence (Met-PRT410) set forth in SEQ ID NO: 7 with VLs. The amino acid sequence set forth in SEQ ID NO: 26 is obtained by substituting all QQs in the amino acid sequence set forth in SEQ ID NO: 7 with TSs, and substituting the remaining Qs with As. The amino acid sequence set forth in SEQ ID NO: 27 is obtained by substituting all QQs in the amino acid sequence set forth in SEQ ID NO: 7 with VLs, and substituting the remaining Qs with Is. The amino acid sequence set forth in SEQ ID NO: 28 is obtained by substituting all QQs in the amino acid sequence set forth in SEQ ID NO: 7 with VIs, and substituting the remaining Qs with Ls. The amino acid sequence set forth in SEQ ID NO: 29 is obtained by substituting all QQs in the amino acid sequence set forth in SEQ ID NO: 7 with VFs, and substituting the remaining Qs with Is.

The amino acid sequence set forth in SEQ ID NO: 30 is obtained by substituting all QQs in the amino acid sequence (Met-PRT525) set forth in SEQ ID NO: 8 with VLs. The amino acid sequence set forth in SEQ ID NO: 31 is obtained by substituting all QQs in the amino acid sequence set forth in SEQ ID NO: 8 with VLs, and substituting the remaining Qs with Is.

The amino acid sequence set forth in SEQ ID NO: 32 is a sequence obtained by substituting all QQs in a sequence obtained by repeating twice a region of 20 domain sequences present in the amino acid sequence (Met-PRT410) set forth in SEQ ID NO: 7 with VFs, and substituting the remaining Qs with Is.

The amino acid sequence (Met-PRT917) set forth in SEQ ID NO: 41 is an amino acid sequence in which all QQs are substituted with LIs and the remaining Qs are substituted with Vs in the amino acid sequence set forth in SEQ ID NO: 7. The amino acid sequence (Met-PRT1028) set forth in SEQ ID NO: 42 is an amino acid sequence in which all QQs are substituted with IFs and the remaining Qs are substituted with Ts in the amino acid sequence set forth in SEQ ID NO: 7.

The glutamine residue content rate of any of the amino acid sequences set forth in SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 41, and SEQ ID NO: 42 is 9% or less (Table 2).

[Table 2]

| Modified fibroin | Glutamine residue content rate | GPGXX motif content rate | Degree of hydrophobicity of REP |
|---|---|---|---|
| Met-PRT410 (SEQ ID NO: 7) | 17.7% | 27.9% | -1.52 |
| Met-PRT888 (SEQ ID NO: 25) | 6.3% | 27.9% | -0.07 |
| Met-PRT965 (SEQ ID NO: 26) | 0.0% | 27.9% | -0.65 |
| Met-PRT889 (SEQ ID NO: 27) | 0.0% | 27.9% | 0.35 |
| Met-PRT916 (SEQ ID NO: 28) | 0.0% | 27.9% | 0.47 |
| Met-PRT918 (SEQ ID NO: 29) | 0.0% | 27.9% | 0.45 |
| Met-PRT699 (SEQ ID NO: 30) | 3.6% | 26.4% | -0.78 |
| Met-PRT698 (SEQ ID NO: 31) | 0.0% | 26.4% | -0.03 |
| Met-PRT966 (SEQ ID NO: 32) | 0.0% | 28.0% | 0.35 |
| Met-PRT917 (SEQ ID NO: 41) | 0.0% | 27.9% | 0.46 |
| Met-PRT1028 (SEQ ID NO: 42) | 0.0% | 28.1% | 0.05 |

The modified fibroin (6-i) may be a modified fibroin consisting of the amino acid sequence set forth in SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 41, or SEQ ID NO: 42.

The modified fibroin (6-ii) may be a modified fibroin containing an amino acid sequence having 90% or more sequence identity with the amino acid sequence set forth in SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 41, or SEQ ID NO: 42. The modified fibroin (6-ii) also is a protein containing a domain sequence represented by Formula 1: $[(A)_n \text{ motif-REP}]_m$ or Formula 2: $[(A)_n \text{ motif-REP}]_m\text{-}(A)_n$ motif. The sequence identity is preferably 95% or more.

The modified fibroin (6-ii) preferably has a glutamine residue content rate of 9% or less. In addition, the modified fibroin (6-ii) preferably has a GPGXX motif content rate of 10% or more.

The sixth modified fibroin may contain a tag sequence at either or both of the N-terminal and C-terminal. This makes it possible to isolate, immobilize, detect, and visualize the modified fibroin.

More specific examples of the modified fibroin containing a tag sequence include a modified fibroin containing (6-iii) the amino acid sequence set forth in SEQ ID NO: 33 (PRT888), SEQ ID NO: 34 (PRT965), SEQ ID NO: 35 (PRT889), SEQ ID NO: 36 (PRT916), SEQ ID NO: 37. (PRT918), SEQ ID NO: 38 (PRT699), SEQ ID NO: 39 (PRT698), SEQ ID NO: 40 (PRT966), SEQ ID NO: 43 (PRT917), or SEQ ID NO: 44 (PRT1028), or a modified fibroin containing (6-iv) an amino acid sequence having 90% or more sequence identity with the amino acid sequence set forth in SEQ ID NO: 33, SEQ ID NO: 34, SEQ ID NO: 35, SEQ ID NO: 36, SEQ ID NO: 37, SEQ ID NO: 38, SEQ ID NO: 39 or SEQ ID NO: 40, SEQ ID NO: 43, or SEQ ID NO: 44.

The amino acid sequences set forth in SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 43, and SEQ ID NO: 44 are amino acid sequences in which the amino acid sequence (containing a His tag sequence and a hinge sequence) set forth in SEQ ID NO: 11 is added to the N-terminal of the amino acid sequences set forth in SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 41, and SEQ ID NO: 42, respectively. Since only the tag sequence is added to the N-terminal, the glutamine residue content rate is not changed, and any of the amino acid sequences set forth in SEQ ID NO:33, SEQ ID NO:34, SEQ ID NO:35, SEQ ID NO:36, SEQ ID NO:37, SEQ ID NO:38, SEQ ID NO:39, SEQ ID NO: 40, SEQ ID NO: 43, and SEQ ID NO: 44 has a glutamine residue content rate of 9% or less (Table 3).

[Table 3]

| Modified fibroin | Glutamine residue content rate | GPGXX motif content rate | Degree of hydrophobicity of REP |
|---|---|---|---|
| PRT888 (SEQ ID NO: 33) | 6.3% | 27.9% | -0.07 |
| PRT965 (SEQ ID NO: 34) | 0.0% | 27.9% | -0.65 |
| PRT889 (SEQ ID NO: 35) | 0.0% | 27.9% | 0.35 |
| PRT916 (SEQ ID NO: 36) | 0.0% | 27.9% | 0.47 |
| PRT918 (SEQ ID NO: 37) | 0.0% | 27.9% | 0.45 |
| PRT699 (SEQ ID NO: 38) | 3.6% | 26.4% | -0.78 |
| PRT698 (SEQ ID NO: 39) | 0.0% | 26.4% | -0.03 |
| PRT966 (SEQ ID NO: 40) | 0.0% | 28.0% | 0.35 |
| PRT917 (SEQ ID NO: 43) | 0.0% | 27.9% | 0.46 |
| PRT1028 (SEQ ID NO: 44) | 0.0% | 28.1% | 0.05 |

The modified fibroin (6-iii) may be a modified fibroin consisting of the amino acid sequence set forth in SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 43, or SEQ ID NO: 44.

The modified fibroin (6-iv) is a modified fibroin containing an amino acid sequence having 90% or more sequence identity with the amino acid sequence set forth in SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 43, or SEQ ID NO: 44. The modified fibroin (6-iv) also is a protein containing a domain sequence represented by Formula 1: $[(A)_n$ motif-REP$]_m$ or Formula 2: $[(A)_n$ motif-REP$]_m$-$(A)_n$ motif. The sequence identity is preferably 95% or more.

The modified fibroin (6-iv) preferably has a glutamine residue content rate of 9% or less. In addition, the modified fibroin (6-iv) preferably has a GPGXX motif content rate of 10% or more.

The sixth modified fibroin may contain a secretory signal for releasing the protein produced in a recombinant protein production system to the outside of the host. The sequence of the secretory signal may be set as appropriate, depending on the type of the host.

The modified fibroin may be a modified fibroin simultaneously pertaining at least two or more characteristics among the characteristics of the first modified fibroin, the second modified fibroin, the third modified fibroin, the fourth modified fibroin, the fifth modified fibroin, and the sixth modified fibroin.

From the viewpoint of securing excellent flame retardancy, the modified fibroin may be a hydrophilic modified fibroin. The hydrophilic modified fibroin is preferably, for example, a modified fibroin in which a value (average HI) obtained by obtaining a total sum of hydropathy indices (HI) of all amino acid residues constituting the modified fibroin, and then dividing the total sum by the total number of amino acid residues is 0 or less. The HI is as shown in Table 1.

Examples of the hydrophilic modified fibroin include a modified fibroin containing the amino acid sequence set forth in SEQ ID NO: 4, the amino acid sequence set forth in SEQ ID NO: 6, SEQ ID NO: 7, SEQ ID NO: 8, or SEQ ID NO: 9, the amino acid sequence set forth in SEQ ID NO: 13, SEQ ID NO: 11, SEQ ID NO: 14, or SEQ ID NO: 15, the amino acid sequence set forth in SEQ ID NO: 18, SEQ ID NO: 7, SEQ ID NO: 8, or SEQ ID NO: 9, the amino acid sequence set forth in SEQ ID NO: 17, SEQ ID NO: 11, SEQ ID NO: 14, or SEQ ID NO: 15, or the amino acid sequence set forth in SEQ ID NO: 19, SEQ ID NO: 20, or SEQ ID NO: 21.

The LOI value of the modified fibroin according to the present embodiment may be 18 or more, 20 or more, 22 or more, 24 or more, 26 or more, 28 or more, 29 or more, or 30 or more. In the present specification, the LOI value is a value measured in accordance with "Test Method for Granular or Low-Melting-Point Synthetic Resin" described in "Notice No. 50 of the Office of Hazardous Materials Regulation (as of May 31, 1995)".

The modified fibroin may be a hydrophobic modified fibroin from the viewpoint of obtaining high hygroscopic exothermicity. The hydrophobic modified fibroin is preferably, for example, a modified fibroin having an average HI of more than 0.

Examples of the hydrophobic modified fibroin include a modified fibroin containing the amino acid sequence set forth in SEQ ID NO: 27, SEQ ID NO: 28, SEQ ID NO: 29, SEQ ID NO: 30, SEQ ID NO: 31, SEQ ID NO: 32, SEQ ID NO: 33, or SEQ ID NO: 43, or the amino acid sequence of SEQ ID NO: 35, SEQ ID NO: 37, SEQ ID NO: 38, SEQ ID NO: 39, SEQ ID NO: 40, SEQ ID NO: 41, or SEQ ID NO: 44.

The maximum hygroscopic heat generation of the modified fibroin according to the present embodiment, which is determined according to Formula A, may be more than 0.025°C/g.

Maximum hygroscopic heat generation = {(Highest temperature of a sample when the sample has been transferred to a high humidity environment after being placed in a low humidity environment until a temperature of the sample reaches equilibrium) - (Temperature of the sample when the sample is being transferred to the high humidity environment after being placed in the low humidity environment until the temperature of the sample reaches equilibrium)} (°C) / Sample weight (g)     Formula A:

Furthermore, in Formula A, the low humidity environment means an environment of a temperature of 20°C and a relative humidity of 40%, and the high humidity environment means an environment of a temperature of 20°C and a relative humidity of 90%.

The maximum hygroscopic heat generation of the modified fibroin according to the present embodiment is 0.026°C/g or more, 0.027°C/g or more, 0.028°C/g or more, 0.029°C/g or more, 0.030°C/g or more, 0.035°C/g or more, or 0.040°C/g or more. The upper limit of the maximum hygroscopic heat generation is not particularly limited, but is usually 0.060°C/g or less.

(Method for Manufacturing Modified Fibroin)

The modified fibroin (hereinafter also simply referred to as a "protein") according to any of the embodiments may also be produced, for example, by expressing a nucleic acid by a host transformed with an expression vector having the nucleic acid sequence encoding the protein and one or a plurality of regulatory sequences operably linked to the nucleic acid sequence.

A method for producing a nucleic acid encoding a modified fibroin is not particularly limited. For example, the nucleic acid may be produced by a method in which a gene encoding a natural fibroin is cloned by amplification with a polymerase chain reaction (PCR) or the like, and subjected to a modification by a genetic engineering technique, or a method of chemically synthesizing a nucleic acid. The method for chemically synthesizing a nucleic acid is not particularly limited, and for example, a gene may be chemically synthesized by a method in which oligonucleotides are automatically synthesized by AKTA oligopilot plus 10/100 (GE Healthcare Japan Corporation) or the like, and are linked by PCR or the like, based on the amino acid sequence information of a protein obtained from the NCBI web database or the like. At this time, in order to facilitate purification and/or confirmation of the modified fibroin, a nucleic acid encoding a modified fibroin consisting of an amino acid sequence obtained by adding an amino acid sequence consisting of a start codon and a His10 tag to the N-terminal of the above-described amino acid sequence may be synthesized.

The regulatory sequence is a sequence (for example, a promoter, an enhancer, a ribosome binding sequence, or a transcription termination sequence) that controls the expression of a modified fibroin in a host, and may be selected as appropriate, depending on the type of the host. As a promoter, an inducible promoter that functions in a host cell and is capable of inducing the expression of a modified fibroin may be used. The inducible promoter is a promoter that can control transcription by the presence of an inducer (expression inducer), the absence of a repressor molecule, or physical factors such as an increase or decrease in a temperature, an osmotic pressure, or a pH value.

The type of the expression vector such as a plasmid vector, a viral vector, a cosmid vector, a fosmid vector, or an artificial chromosome vector may be selected as appropriate, depending on the type of the host. As the expression vector, an expression vector that can autonomously replicate in a host cell or can be incorporated into a chromosome of a host and which contains a promoter at a position capable of transcribing the nucleic acid that encodes a modified fibroin is suitably used.

Both prokaryotes and eukaryotes such as yeast, filamentous fungi, insect cells, animal cells, and plant cells may be suitably used as a host.

Preferred examples of the prokaryotic host cells include bacteria belonging to the genus Escherichia, the genus Brevibacillus, the genus Serratia, the genus Bacillus, the genus Microbacterium, the genus Brevibacterium, the genus Corynebacterium, and the genus Pseudomonas. Examples of microorganisms belonging to the genus Escherichia include Escherichia coli. Examples of microorganisms belonging to the genus Brevibacillus include Brevibacillus agri. Examples of microorganisms belonging to the genus Serratia include Serratia liquefaciens. Examples of microorganisms belonging to the genus Bacillus include Bacillus subtilis. Examples of microorganisms belonging to the genus Microbacterium include Microbacterium ammoniaphilum. Examples of microorganisms belonging to the genus Brevibacterium include Brevibacterium divaricatum. Examples of microorganisms belonging to the genus Corynebacterium include Corynebacterium ammoniagenes. Examples of microorganisms belonging to the genus Pseudomonas include Pseudomonas putida.

In a case where a prokaryote is used as a host, examples of a vector into which a nucleic acid encoding a modified fibroin is introduced include pBTrp2 (manufactured by Boehringer Mannheim), pGEX (manufactured by Pharmacia), pUC18, pBluescriptII, pSupex, pET22b, pCold, pUB110, and pNCO2 (Japanese Unexamined Patent Publication No. 2002-238569).

Examples of the eukaryotic hosts include yeast and filamentous fungi (mold and the like). Examples of the yeasts include yeasts belonging to the genus Saccharomyces, the genus Pichia, and the genus Schizosaccharomyces. Examples of the filamentous fungi include filamentous fungi belonging to the genus Aspergillus, the genus Penicillium, and the genus Trichoderma.

In a case where the eukaryote is used as a host, examples of the vector into which a nucleic acid encoding a modified fibroin is introduced include YEp13 (ATCC37115) and YEp24 (ATCC37051). As a method for introducing an expression vector into the host cell, any of methods of introducing DNA into the host cell may be used. Examples thereof include a method using calcium ions [Proc. Natl. Acad. Sci. USA, 69, 2110 (1972)], electroporation method, spheroplast method, protoplast method, lithium acetate method, and competent method.

As for the method for expressing a nucleic acid using a host transformed with an expression vector, secretory production, fusion protein expression, or the like, in addition to the direct expression, may be performed in accordance with the method described in Molecular Cloning, 2nd edition, and the like.

The modified fibroin may be produced, for example, by culturing a host transformed with the expression vector in a culture medium, producing and accumulating the protein in the culture medium, and then collecting the modified fibroin from the culture medium. The method for culturing a host in a culture medium may be performed in accordance with a method commonly used for culturing a host.

In a case where the host is a prokaryote such as Escherichia coli or a eukaryote such as yeast, any of a natural medium and a synthetic medium may be used as a culture medium of the host as long as the medium contains a carbon source, a nitrogen source, inorganic salts, and the like which may be utilized by the host and the medium may be used for efficiently culturing the host.

The carbon source may be any of carbon sources which can be assimilated by a transformed microorganism, and for example, carbohydrates such as glucose, fructose, sucrose, and molasses, starch, and starch hydrolysates containing these, organic acids such as acetic acid and propionic acid, and alcohols such as ethanol and propanol may be used. As the nitrogen source, for example, ammonium salts of inorganic or organic acids such as ammonia, ammonium chloride, ammonium sulfate, ammonium acetate, and ammonium phosphate, other nitrogen-containing compounds, peptone, meat extract, yeast extract, corn steep liquor, casein hydrolysate, soybean cake and soybean cake hydrolysate, and various fermented microbial cells and digested products thereof may be used. As the inorganic salts, for example, potassium dihydrogen phosphate, dipotassium phosphate, magnesium phosphate, magnesium sulfate, sodium chloride, ferrous sulfate, manganese sulfate, copper sulfate, and calcium carbonate may be used.

Culture of a prokaryote such as Escherichia coli or a eukaryote such as a yeast may be performed under aerobic conditions such as shaking culture or deep aeration stirring culture. The culture temperature is, for example, 15°C to 40°C. The culture time is usually 16 hours to 7 days. It is preferable to maintain the pH of the culture medium during the culture at 3.0 to 9.0. The pH of the culture medium may be adjusted using an inorganic acid, an organic acid, an alkali solution, urea, calcium carbonate, ammonia, or the like.

In addition, antibiotics such as ampicillin and tetracycline may be added to the culture medium as necessary during the culture. In a case of culturing a microorganism transformed with an expression vector using an inducible promoter as the promoter, an inducer may be added to the medium as necessary. For example, in a case of culturing a microorganism transformed with an expression vector using a lac promoter, isopropyl-$\beta$-D-thiogalactopyranoside or the like may be added to the medium, and in a case of culturing a microorganism transformed with an expression vector using a trp promoter, indole acrylic acid or the like may be added to the medium.

Isolation and purification of the expressed modified fibroin may be performed by a commonly used method. For example, in a case where the protein is expressed in a dissolved state in cells, the host cells are recovered by centrifugation after the completion of the culture, suspended in an aqueous buffer solution, and then disrupted using an ultrasonicator, a French press, a Manton-Gaulin homogenizer, a Dyno-Mill, or the like to obtain a cell-free extract. From the supernatant obtained by centrifuging the cell-free extract, a purified preparation may be obtained by a method commonly used for protein isolation and purification, that is, a solvent extraction method, a salting-out method using ammonium sulfate or the like, a desalting method, a precipitation method using an organic solvent, an anion exchange chromatography method using a resin such as diethylaminoethyl (DEAE)-Sepharose or DIAION HPA-75 (manufactured by Mitsubishi Kasei Kogyo Kabushiki Kaisha), an cation exchange chromatography method using a resin such as S-Sepharose FF (manufactured by Pharmacia Corporation), a hydrophobic chromatography method using a resin such as butyl sepharose or phenyl sepharose, a gel filtration method using a molecular sieve, an affinity chromatography method, a chromatofocusing method, or an electrophoresis method such as isoelectric focusing or the like, using the above-mentioned methods singly or in combination thereof.

In addition, in a case where the modified fibroin is expressed to form an insoluble body in the cell, similarly, the host cells are recovered, disrupted, and centrifuged to recover the insoluble body of the modified fibroin as a precipitated fraction. The recovered insoluble body of the modified fibroin may be solubilized with a protein denaturing agent. After this operation, a purified preparation of the modified fibroin may be obtained by the same isolation and purification method as described above. In a case where the protein is secreted extracellularly, the protein may be recovered from the culture supernatant. That is, a culture supernatant is obtained by treating the culture by a technique such as centrifugation, and a purified preparation may be obtained from the culture supernatant by using the same isolation and purification method as described above.

(Method for Manufacturing Modified Fibroin Fiber)

In a case where the modified fibroin fiber is a modified fibroin filament, the modified fibroin filament may be manufactured by a known spinning method. That is, for example, in a case of manufacturing a modified fibroin filament containing a modified fibroin as the main component, first, the modified fibroin produced in accordance with the above-mentioned method is added to a solvent such as dimethyl sulfoxide (DMSO), N,N-dimethylformamide (DMF), formic acid, or hexafluoroisopropanol (HFIP), as necessary, together with an inorganic salt as a dissolution accelerator and dissolved to prepare a doping liquid. Then, using the doping liquid, spinning may be performed by a known spinning method such as wet-type spinning, dry-type spinning, dry-wet-type spinning, or melt spinning to obtain the modified fibroin filament. Preferred examples of the spinning method include wet-type spinning and dry-wet-type spinning.

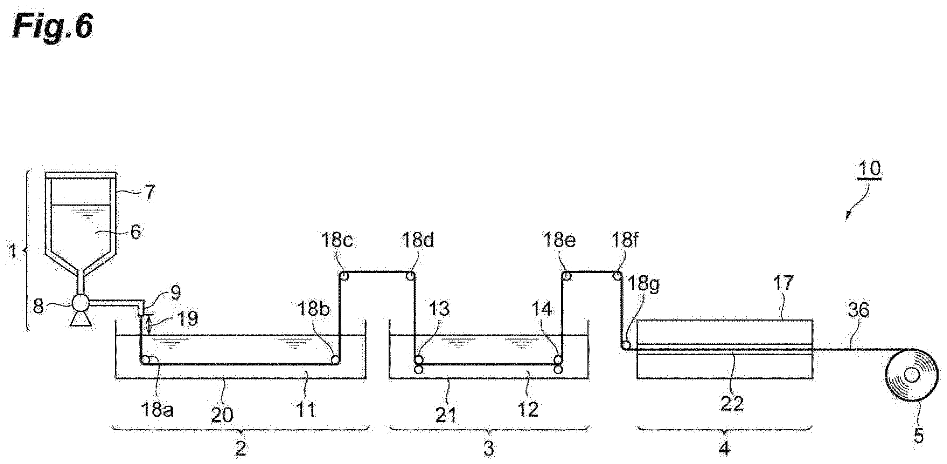

Fig. 6 is an explanatory view schematically illustrating one example of a spinning device for manufacturing a modified fibroin filament. A spinning device 10 shown in Fig. 6 is an example of a spinning device for dry-wet-type spinning, and includes an extrusion device 1, an undrawn yarn manufacturing device 2, a wet heat drawing device 3, and a drying device 4.

A spinning method using the spinning device 10 will be described. First, a doping liquid 6 stored in a storage tank 7 is pushed out from a spinneret 9 by a gear pump 8. In the laboratory scale, the doping liquid may be filled in a cylinder and extruded from a nozzle using a syringe pump. Next, the extruded doping liquid 6 is supplied into a coagulation liquid 11 in a coagulation liquid bath 20 via an air gap 19, the solvent is removed, the modified fibroin is coagulated, and a fibrous coagulate is formed. Then, the fibrous coagulate is supplied into warm water 12 in a drawing bath 21 and is drawn. A drawing ratio is determined according to a speed ratio of a supply nip roller 13 to a withdrawing nip roller 14. Thereafter, the drawn fibrous coagulate is supplied to a drying device 4 and dried in a yarn path 22 to obtain a modified fibroin filament 36 as a wound yarn body 5. Reference signs 18a to 18g are yarn guides.

The coagulation liquid 11 may be any solvent that can be desolvated, and examples thereof include lower alcohols having 1 to 5 carbon atoms such as methanol, ethanol, and 2-propanol, and acetone. The coagulation liquid 11 may include water as appropriate. The temperature of the coagulation liquid 11 is preferably 0°C to 30°C. In a case where a syringe pump having a nozzle with a diameter of 0.1 to 0.6 mm is used as the spinneret 9, the extrusion speed is preferably 0.2 to 6.0 mL/hour, and more preferably 1.4 to 4.0 mL/hour, per hole. A distance that the coagulated modified fibroin passes through the coagulation liquid 11 (substantially a distance from the yarn guide 18a to the yarn guide 18b) may be a length that allows efficient desolvation, and is, for example, 200 to 500 mm. The withdrawing speed of the undrawn yarn may be, for example, 1 to 20 m/min, and is preferably 1 to 3 m/min. The residence time in the coagulation liquid 11 may be, for example, 0.01 to 3 minutes, and is preferably 0.05 to 0.15 minutes. In addition, drawing (pre-drawing) may be performed in the coagulation liquid 11. The coagulation liquid bath 20 may be provided in multiple stages, and the drawing may be performed in each stage or in a specific stage as necessary.

Moreover, as the drawing to be carried out in a case of obtaining a modified fibroin filament, for example, a pre-drawing performed in the coagulation liquid bath 20 and a wet heat drawing performed in the drawing bath 21 are employed, and a dry heat drawing is also employed.

The wet heat drawing may be performed in warm water, in a solution obtained by adding an organic solvent or the like to warm water, or in heated steam. The temperature may be, for example, 50°C to 90°C, and is preferably 75°C to 85°C. In the wet heat drawing, the undrawn yarn (or pre-drawn yarn) may be drawn, for example, by 1 to 10 times, and preferably by 2 to 8 times.

The dry heat drawing may be performed using an electric tubular furnace, a dry heat plate, or the like. The temperature may be, for example, 140°C to 270°C, and is preferably 160°C to 230°C. In the dry heat drawing, the undrawn yarn (or pre-drawn yarn) may be drawn, for example, by 0.5 to 8 times, and is preferably drawn by 1 to 4 times.

The wet heat drawing and the dry heat drawing may be performed independently or in combination, or may be performed in multiple stages. That is, the wet heat drawing and the dry heat drawing may be performed in a suitable combination, for example, in a manner in which a first stage drawing is performed by wet heat drawing and a second stage drawing is performed by dry heat drawing or in a manner in which the first stage drawing is performed by wet heat drawing, the second stage drawing is performed by wet heat drawing, and a third stage drawing is performed by dry heat drawing.

The lower limit value of the final drawing ratio with respect to the undrawn yarn (or pre-drawn yarn) is preferably any of more than 1 time, 2 times or more, 3 times or more, 4 times or more, 5 times or more, 6 times or more, 7 times or more, 8 times or more, or 9 times or more, and the upper limit value is preferably 40 times or less, 30 times or less, 20 times or less, 15 times or less, 14 times or less, 13 times or less 12 times or less, 11 times or less, or 10 times or less.

The length of the modified fibroin filament obtained by the above-mentioned method may be adjusted by the spinning conditions as appropriate. The length of the modified fibroin filament is not particularly limited, but may be, for example, more than 1,500 m, and may be 10,000 m or more, 15,000 m or more, or 20,000 m or more.

(Cutting Step)

In a case where the modified fibroin fiber is a modified fibroin staple, the modified fibroin staple may be manufactured by cutting a modified fibroin filament produced by the above-described method. Further, cutting of the modified fibroin filament may be performed after a crimping step or after a drying step, which will be described later. The length of the modified fibroin staple is not particularly limited, but may be 20 mm or more, 20 to 140 mm, 70 to 140 mm, or 20 to 70 mm.

(Crimping Step)

In a case where the modified fibroin fiber is a modified fibroin crimped fiber (modified fibroin crimped staple or modified fibroin crimped staple), the modified fibroin crimped fiber may be produced, for example, by crimping a non-crimped modified fibroin fiber. The modified fibroin fiber may be crimped by a mechanical crimping process in the related art or may be crimped by being brought into contact with an aqueous medium. In addition, these processing methods may be used in combination.

Examples of the mechanical crimping processing method include a false twist method, an indentation method, a rubbing method, an air jet method (high-pressure air jet method), and a shaping method.

The modified fibroin fiber in the disclosure shrinks by being brought into contact with water, and is thus crimped. Therefore, according to the processing method of bringing the modified fibroin fiber into contact with the aqueous medium, the modified fibroin fiber can be crimped without depending on an external force. The aqueous medium is a medium of a liquid or gas (steam) including water (including a water vapor). The aqueous medium may be water or may be a mixed liquid of water and a hydrophilic solvent. Further, as the hydrophilic solvent, for example, a volatile solvent such as ethanol and methanol or a vapor thereof may be used. The aqueous medium is preferably a mixed solution of water and ethanol. By using an aqueous medium including a volatile solvent or a vapor thereof, the modified fibroin fiber dries quickly, and is finished to have a soft feel. The ratio of water to the volatile solvent or a vapor thereof is not particularly limited, and water:the volatile solvent or a vapor thereof may be, for example, 10:90 to 90:10 in terms of a mass ratio.

In a case where the aqueous medium is a liquid, a known oil agent such as an oil agent for process passability (for example, for antistatic properties) or for finishing may be dispersed in the aqueous medium. That is, instead of the aqueous medium, an oil agent dispersion liquid including an aqueous medium and an oil agent dispersed in the aqueous medium may be used. By using such an oil agent dispersion liquid, the modified fibroin fiber may be crimped, and the oil agent can be attached to the modified fibroin fiber. By attaching the oil agent to the modified fibroin fiber, various properties can be imparted to the modified fibroin fiber. Further, the amount of the oil agent is not particularly limited and may be, for example, 1% to 10% by mass or 2% to 5% by mass with respect to the total amount of the aqueous medium and the oil agent.

The temperature of the aqueous medium may be 10°C or higher, 25°C or higher, 40°C or higher, 60°C or higher, or 100°C or higher, and may be 230°C or lower, 120°C or lower, or 100°C or lower. More specifically, in a case where the aqueous medium is a gas (steam), the temperature of the aqueous medium is preferably 100°C to 230°C, and more preferably 100°C to 120°C. In a case where the steam of the aqueous medium is 230°C or lower, the heat denaturation of the modified fibroin fiber can be prevented. In a case where the aqueous medium is a liquid, the temperature of the aqueous medium is preferably 10°C or higher, 25°C or higher, or 40°C or higher from the viewpoint of efficiently imparting crimpness, and is preferably 60°C or lower from the viewpoint of maintaining a high fiber strength of the modified fibroin fiber. The time of bringing the modified fibroin fiber into contact with the aqueous medium is not particularly limited, but may be 30 seconds or longer, 1 minute or longer, or 2 minutes or longer, and is preferably 10 minutes or shorter from the viewpoint of productivity. The contact of the aqueous medium with the modified fibroin fiber may be performed under normal pressure or under reduced pressure (for example, vacuum).

Examples of a method of bringing the modified fibroin fiber into contact with the aqueous medium include a method of immersing a modified fibroin fiber in water, a method of spraying steam of an aqueous medium onto a modified fibroin fiber, and a method of exposing a modified fibroin fiber to an environment filled with steam in an aqueous medium. In a case where the aqueous medium is steam, a contact of the aqueous medium with the modified fibroin fiber may be performed using a general steam setting device. Specific examples of the steam setting device include a device of product name: FMSA type steam setter (manufactured by Fukushin Kyougyo Co., Ltd.) and a device of a product name: EPS-400 (manufactured by Tsujii Dyeing Machine Manufacturing Co., Ltd.). Specific examples of the method for crimping the modified fibroin fiber with the steam of the aqueous medium include a method of accommodating a modified fibroin fiber in a predetermined accommodating chamber and also bringing the modified fibroin fiber into contact with steam while introducing the steam of an aqueous medium into the accommodating chamber to adjust the temperature inside the accommodating chamber to a predetermined temperature (for example, 100°C to 230°C).

Moreover, the crimping step of the modified fibroin fiber by the contact with the aqueous medium is preferably carried out in a state where no tensile force is applied to a modified fibroin fiber (not tensioned in the fiber axis direction) or in a state where a predetermined amount of tensile force is applied (tensioned by a predetermined amount in the fiber axis direction). At this time, the degree of crimpness may be controlled by adjusting the tensile force applied to the modified fibroin fiber. Examples of the method for adjusting the tensile force applied to the modified fibroin fiber include a method of adjusting a load applied to a modified fibroin fiber, for example, by hanging weight units of various weights on the modified fibroin fiber, and the like, a method of fixing both ends of a modified fibroin fiber in a state where the modified fibroin fiber is slackened while changing the amount of slackness in various ways, and a method of winding a modified fibroin fiber around a wound body such as a paper tube or a bobbin so that a winding force is changed as appropriate, (a tightening force on the paper tube or the bobbin).

(Drying Step)

After bringing the modified fibroin fiber into contact with the aqueous medium, the modified fibroin fiber may be dried. The drying method is not particularly limited, and the modified fibroin fiber may be naturally dried or may be forcibly dried using a drying facility. Crimping with the aqueous medium and subsequent drying may be continuously performed. Specifically, for example, the modified fibroin fiber may be dried by immersing the modified fibroin fiber in the aqueous medium while sending out the modified fibroin fiber from a bobbin, and then blowing hot air or sending the modified fibroin fiber out on a hot roller. The drying temperature is not particularly limited, may be, for example, 20°C to 150°C, and is preferably 40°C to 120°C, and more preferably 60°C to 100°C.

<Knitted or Woven Body>

One aspect of the present invention relates to a knitted or woven body of the blended yarn. The knitted or woven body is a generic term for a knitted fabric and a woven fabric. The blended yarn constituting the knitted or woven body may be a single yarn or may be a double yarn. The knitted fabric may be any of a knitted fabric having a weft knitted structure (also simply referred to as a "weft knitted fabric") such as a flat knit and a circular knit, and a knitted fabric having a warp knitted structure (also simply referred to as a "warp knitted fabric") such as tricot and russell. The woven fabric may be a woven fabric having any structure of a plain weave, a twill weave, and a satin weave. The knitted or woven body may be an unprocessed knitted or woven body itself obtained by knitting or weaving, or may be a knitted or woven body which has been subjected to a process such as a shrinking process after knitting or weaving.

<Method for Manufacturing Knitted or Woven Body>

The knitted or woven body according to the present invention is manufactured by a method including steps of: blend-spinning a modified fibroin fiber and a regenerated cellulose fiber to obtain a blended yarn (blend-spinning step); and knitting or weaving the blended yarn to obtain an unprocessed knitted or woven body (knitting or weaving step).

(Blend-Spinning Step)

In the present step, a modified fibroin fiber and a regenerated cellulose fiber are blend-spun to obtain a blended yarn. Details of the modified fibroin fiber, the regenerated cellulose fiber, and the blended yarn are as described above. The blend-spinning method is not particularly limited and a known spinning method may be adopted.

(Knitting or Weaving Step)

In the present step, the blended yarn is knitted or woven to obtain an unprocessed knitted or woven body (an unprocessed knitted fabric or an unprocessed woven fabric). As the knitting method and the weaving method, known methods may be used. As a knitting machine to be used, for example, a circular knitting machine, a warp knitting machine, a flat knitting machine, or the like may be used, and from the viewpoint of productivity, the circular knitting machine is preferably used. As a flat knitting machine, there are a shaped knitting machine, a seamless knitting machine, and the like, but in particular, the use of a seamless knitting machine is more preferable since an unprocessed knitted fabric may be manufactured in the form of a final product. Examples of a weaving machine to be used include a shuttle weaving machine, and a shuttleless weaving machine such as a gripper weaving machine, a rapier weaving machine, a water jet weaving machine, and an air jet weaving machine.

(Shrinking Step)

The obtained unprocessed knitted or woven body may be optionally subjected to various types of processing. An example of the processing is a processing in which an unprocessed knitted or woven body is brought into contact with an aqueous medium to cause a modified fibroin fiber contained in the unprocessed knitted or woven body to shrink. This processing may be carried out by a method similar to the method of crimping a modified fibroin fiber by bringing the modified fibroin fiber into contact with an aqueous medium. That is, the modified fibroin fiber contained in the unprocessed knitted or woven body may be shrunk by a method such as a method of immersing an unprocessed knitted or woven body in water, a method of spraying steam of an aqueous medium onto an unprocessed knitted or woven body, a method of exposing an unprocessed knitted or woven body to an environment filled with steam of an aqueous medium, or the like, and the details of the aqueous medium and the respective conditions are as described above. More specifically, the modified fibroin fiber contained in the unprocessed knitted or woven body may be shrunk by bringing the unprocessed knitted or woven body into contact with the aqueous medium, followed by drying. The knitted or woven body obtained through shrinking has a high density, and a decrease in tension and stiffness due to moistness is further reduced.

**Examples**

Hereinafter, the present invention will be more specifically described, based on Examples. However, the present invention is not limited to the following Examples.

<Manufacture of Modified Spider Silk Fibroin>

(1) Preparation of Plasmid Expressing Strain

Based on the nucleotide sequence and the amino acid sequence of a fibroin (GenBank Accession No.: P46804.1, GI: 1174415) derived from Nephila clavipes, a modified fibroin having the amino acid sequence set forth in SEQ ID NO: 15 (PRT799) was designed. In addition, a modified fibroin having the amino acid sequence set forth in SEQ ID NO: 37 (PRT918) and a modified fibroin having the amino acid sequence set forth in SEQ ID NO: 40 (PRT966) were also designed.
Next, a nucleic acid encoding the designed modified fibroin was synthesized. In the nucleic acid, an NdeI site was added to the 5' end and an EcoRI site was added downstream of the stop codon. The nucleic acid was cloned into a cloning vector (pUC118). Thereafter, the nucleic acid was cleaved by a restriction enzyme treatment with NdeI and EcoRI, and then recombined into a protein expression vector pET-22b (+) to obtain an expression vector.

(2) Expression of Protein

Escherichia coli BLR(DE3) was transformed with a pET22b(+) expression vector containing the designed nucleic acid encoding a modified fibroin. The transformed Escherichia coli was cultured in 2 mL of an LB medium containing ampicillin for 15 hours. The culture solution was added to 100 mL of a seed culture medium (Table 4) containing ampicillin so that the $OD_{600}$ was 0.005. While maintaining the temperature of the culture solution at 30°C, flask culturing was performed (for about 15 hours) until the $OD_{600}$ reached 5, thereby obtaining a seed culture solution.

[Table 4]

| Seed culture medium | |
|---|---|
| Reagent | Concentration (g/L) |
| Glucose | 5.0 |
| $KH_2PO_4$ | 4.0 |
| $K_2HPO_4$ | 9.3 |
| Yeast Extract | 6.0 |
| Ampicillin | 0.1 |

To a jar fermenter to which 500 mL of a production medium (Table 5) had been added, the seed culture solution was added so that the $OD_{600}$ was 0.05. The culture was performed while keeping the culture solution temperature at 37°C and controlling the pH constant at 6.9. Further, the concentration of dissolved oxygen in the culture solution was maintained at 20% of the dissolved oxygen saturation concentration.

[Table 5]

| Production medium | |
| --- | --- |
| Reagent | Concentration (g/L) |
| Glucose | 12.0 |
| $KH_2PO_4$ | 9.0 |
| $MgSO_4 \cdot 7H_2O$ | 2.4 |
| Yeast Extract | 15 |
| $FeSO_4 \cdot 7H_2O$ | 0.04 |
| $MnSO_4 \cdot 5H_2O$ | 0.04 |
| $CaCl_2 \cdot 2H_2O$ | 0.04 |
| GD-113 (antifoamer) | 0.1 (mL/L) |

Immediately after glucose in the production medium was completely consumed, a feed solution (455 g/1 L of glucose and 120 g/1 L of Yeast Extract) was added at a rate of 1 mL/min. The culture was performed while keeping the culture solution temperature at 37°C and controlling the pH constant at 6.9. Further, the concentration of dissolved oxygen in the culture solution was maintained at 20% of the dissolved oxygen saturation concentration, and the culture was performed for 20 hours. Thereafter, 1 M isopropyl-β-thiogalactopyranoside (IPTG) was added to the culture solution to a final concentration of 1 mM to induce the expression of the modified fibroin. After the lapse of 20 hours from the addition of IPTG, the culture solution was centrifuged to recover the bacterial cell pellet. SDS-PAGE was performed using bacterial cell pellets prepared from the culture solution before the addition of IPTG and after the addition of IPTG, and the expression of the target modified fibroin was checked by the IPTG addition-dependent appearance of a band corresponding to the size of the target modified fibroin.

(3) Purification of Protein

The bacterial cell pellet recovered after 2 hours from the addition of IPTG was washed with a 20 mM Tris-HCl buffer solution (pH 7.4). The bacterial cell pellet after washing was suspended in a 20 mM Tris-HCl buffer solution (pH 7.4) containing about 1 mM PMSF, and the cell suspension was disrupted with a high-pressure homogenizer (manufactured by GEA Niro Soavi SpA). The disrupted cells were centrifuged to obtain a precipitate. The obtained precipitate was washed with a 20 mM Tris-HCl buffer solution (pH 7.4) until the obtained precipitate became highly pure. The precipitate after washing was suspended in an 8 M guanidine buffer solution (8 M guanidine hydrochloride, 10 mM sodium dihydrogen phosphate, 20 mM NaCl, 1 mM Tris-HCl, pH 7.0) so that the concentration was 100 mg/mL, and dissolved by stirring with a stirrer at 60°C for 30 minutes. After dissolution, dialysis was performed in water using a dialysis tube (cellulose tube 36/32 manufactured by Sanko Junyaku Co., Ltd.). The white protein aggregate obtained after dialysis was recovered by centrifugation, the moisture was removed with a lyophilizer, and the lyophilized powder was recovered to obtain modified spider silk fibroins "PRT799", "PRT918", and "PRT966".

<Manufacture of Modified Fibroin Filament>

(1) Preparation of Doping Liquid

The above-described modified fibroin (PRT799, PRT918, or PRT966) was added to DMSO so that the concentration was 24% by mass, and then LiCl was added thereto as a dissolution accelerator so that the concentration was 4.0% by mass. Then, the modified fibroin was dissolved for 3 hours using a shaker to obtain a DMSO solution of the modified fibroin. The insoluble matter and foams in the obtained DMSO solution were removed to obtain a doping liquid. The solution viscosity of the doping liquid was 5,000 centipoises (cP) at 90°C.

(2) Spinning

Known dry-wet-type spinning was performed using the doping liquid obtained as described above and the spinning device 10 shown in Fig. 6 to obtain a modified fibroin filament. The obtained modified fibroin filament was wound on a bobbin. Further, the dry-wet-type spinning was performed here under the following conditions.

Temperature of coagulation liquid (methanol): 5°C to 10°C
Drawing ratio: 4.52 times
Drying temperature: 80°C

<Example>

A plurality of modified fibroin filaments obtained as described above were bundled and cut into a length of 38 mm with a tabletop-type fiber cutting machine to prepare a modified fibroin staple. The modified fibroin staple thus prepared was immersed in water at 40°C for 1 minute to be crimped, and then dried at 40°C for 18 hours to obtain a modified fibroin crimped staple. The shrinkage rate of the modified fibroin staple by immersion in water was 50%. This modified fibroin crimped staple and a commercially available lyocell crimped staple were spun using known spinning equipment to obtain a double yarn of a blended yarn having a fineness of 2/30 Nm (2 / 333 dtex) and a twist number of 570 T/m. The proportion of the modified fibroin crimped staple in the blended yarn was 20% by mass. A knitted fabric was prepared by performing plain knitting with a flat knitting machine, using a double yarn of the blended yarn.

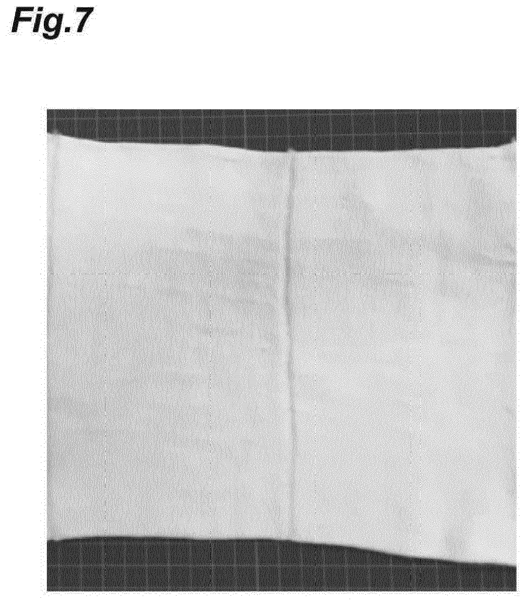

The knitted fabric was immersed in water, and changes in the tension and the stiffness after drying were observed. Specifically, first, the knitted fabric whose weight (initial weight) had been measured in advance was immersed in water at a temperature of 37.5°C. After 90 seconds, the knitted fabric was taken out of water and the moisture of the knitted fabric was removed with a paper towel. The knitted fabric was dried with a dryer (EH5101P manufactured by Panasonic) until the weight returned to the initial weight. A photograph of the knitted fabric after drying is shown in Fig. 7. The knitted fabric after drying was in a slightly thicker and firmer state than before being immersed in water.

<Comparative Example>

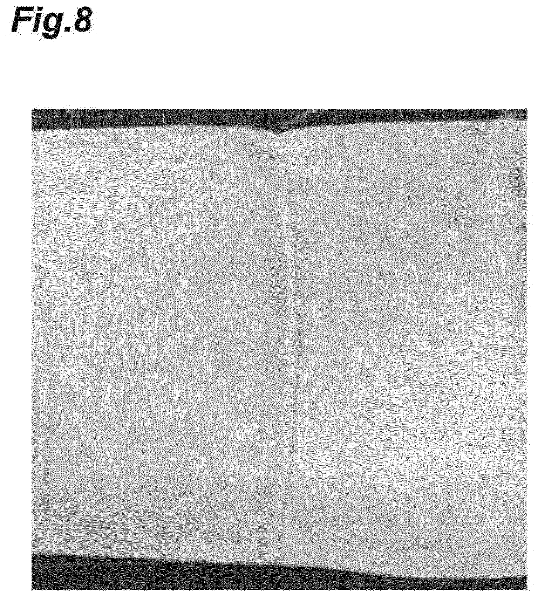

A knitted fabric was prepared by performing plain knitting with a flat knitting machine, using a double yarn of a commercially available lyocell spun yarn having a fineness of 2/30 Nm (2 / 333 dtex) and a twist number of 295 T/m. In a similar manner to the example, the knitted fabric was immersed in water, and changes in the tension and the stiffness after drying were observed. A photograph of the knitted fabric after drying is shown in Fig. 8.

In the knitted fabric of the example, decrease in the tension and the stiffness after immersion in water and drying was suppressed, as compared with the knitted fabric of the comparative example.

<Reference Example 1>

A doping liquid of the modified fibroin PRT799 obtained in the same manner as in Example above was filtered through a metal filter having an opening of 5 μm at 60°C, then allowed to stand in a 30 mL stainless steel syringe, defoamed, and then discharged from a solid nozzle having a needle diameter of 0.2 mm into a 100%-by-mass methanol coagulation bath. The discharge temperature was 60°C. After coagulation, the obtained raw yarn was wound and naturally dried to obtain a modified fibroin fiber.

A knitted fabric was manufactured by circular knitting using a circular knitting machine, using the obtained raw material fiber. The knitted fabric had a yarn thickness of 180 denier (200 dtex) and a gauge number of 18. 20 g of a piece was cut out from the obtained knitted fabric and used as a test piece.

A flammability test was performed in accordance with "Test Method for Granular or Low-Melting-Point Synthetic Resin" described in "Notice No. 50 of the Office of Hazardous Materials Regulation (as of May 31, 1995)". The test was performed under the conditions of a temperature of 22°C, a relative humidity of 45%, and an atmospheric pressure of 1,021 hPa. The measurement results (oxygen concentration (%), combustion rate (%), and converted combustion rate (%)) are shown in Table 6.

[Table 6]

| Oxygen concentration (%) | Combustion rate (%) | Converted combustion rate (%) |
|---|---|---|
| 20.0 | 39.1 | 40.1 |
| 27.0 | 48.1 | 49.3 |
| 28.0 | 51.9 | 53.2 |
| 30.0 | 53.6 | 54.9 |
| 50.0 | 61.2 | 62.7 |
| 70.0 | 91.1 | 93.3 |

(continued)

| Oxygen concentration (%) | Combustion rate (%) | Converted combustion rate (%) |
|---|---|---|
| 100.0 | 97.6 | 100.0 |

As a result of the flammability test, the knitted fabric knitted with the modified fibroin (PRT799) fiber had a limiting oxygen index (LOI) value of 27.2. Generally, LOI value of 26 or more is considered as having flame retardancy. It can be seen that the modified fibroin has an excellent flame retardancy. Therefore, it can be said that a blended yarn containing such a modified fibroin fiber and a knitted or woven body of the same have excellent flame retardancy.

<Reference Example 2>

A doping liquid of the modified fibroin (PRT799 or PRT918) obtained in the same manner as in Example above was filtered through a metal filter having an opening of 5 μm at 60°C, then allowed to stand in a 30 mL stainless steel syringe, defoamed, and then discharged from a solid nozzle having a needle diameter of 0.2 mm into a 100%-by-mass methanol coagulation bath. The discharge temperature was 60°C. After coagulation, the obtained raw yarn was wound and naturally dried to obtain a modified fibroin fiber (raw material fiber).

For comparison, commercially available wool fibers, cotton fibers, tencel fibers, rayon fibers, and polyester fibers were prepared as the raw material fibers.

Each raw material fiber was used to manufacture a knitted fabric by flat knitting using a flat knitting machine. The knitted fabric using the PRT918 fiber as the raw material fiber had a yarn thickness of 1/30 N (single wool count yarn) (295 dtex) and a gauge number of 18. The knitted fabric using the PRT799 fiber as the raw material fiber had a yarn thickness of 1/30 N (single wool count yarn) (295 dtex) and a gauge number of 16. The yarn thickness and the gauge number of the knitted fabric for which such other raw material fibers were used were adjusted so that the cover factor was almost the same as that of the knitted fabric using the PRT918 fiber and the PRT799 fiber. Further, the yarn thickness and the gauge number of a knitted fabric using a fiber other than the modified fibroin fiber were adjusted as follows.

Wool fiber thickness: 2/30 N Z (2 / 295 dtex) (double yarn), gauge number: 14
Cotton fiber thickness: 2/34 N (2 / 260 dtex) (double yarn), gauge number: 14
Tencel fiber thickness: 2/30 N (2 / 295 dtex) (double yarn), gauge number: 15
Rayon fiber thickness: 1/38 N (1 / 233 dtex) (single yarn), gauge number: 14
Polyester fiber thickness: 1/60 N (1 / 148 dtex) (single yarn), gauge number: 14

Two sheets of the knitted fabric cut into 10 cm × 10 cm were put together and the four sides were sewn together to obtain a test piece (sample). The test piece was left to stand in a low humidity environment (temperature of 20°C ± 2°C, relative humidity of 40% ± 5%) for 4 hours or longer and then transferred to a high humidity environment (temperature of 20°C ± 2°C, relative humidity of 90% ± 5%), and measurement of the temperature was performed at an interval of 1 minute for 30 minutes with a temperature sensor mounted at the center of the inside of the test piece.

From the measurement results, the maximum hygroscopic heat generation was obtained according to the following Formula A.

Formula A: Maximum hygroscopic heat generation = {(Highest temperature of a sample when the sample has been transferred to a high humidity environment after being placed in a low humidity environment until a temperature of the sample reaches equilibrium) - (Temperature of --> the sample when the sample is being transferred to the high humidity environment after being placed in the low humidity environment until the temperature of the sample reaches equilibrium)} (°C) / Sample weight (g)        Formula A:

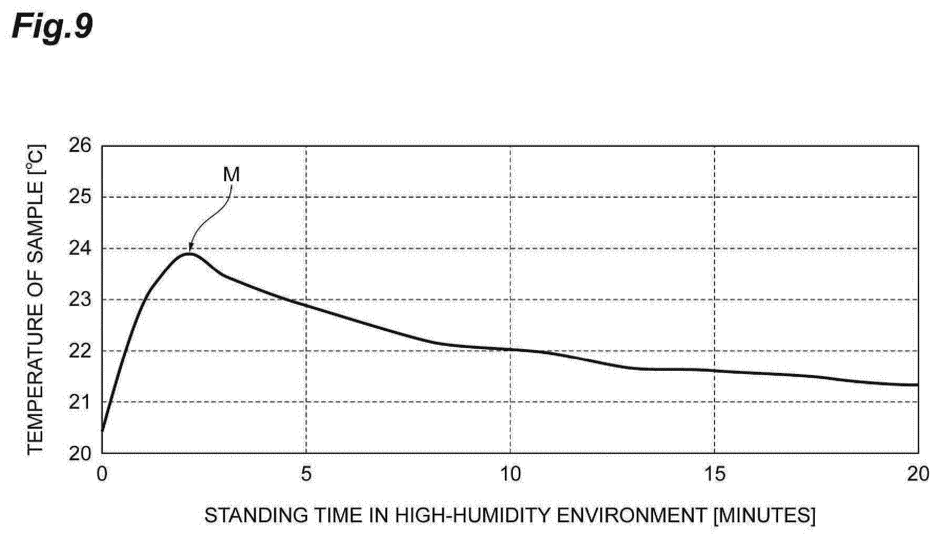

Fig. 9 is a graph illustrating an example of the results of the hygroscopic heat generation test. The horizontal axis of the graph indicates a standing time (minutes) in the high humidity environment, with a point of time of transferring the sample from the low humidity environment to the high humidity environment being set to 0. The vertical axis of the graph indicates a temperature (temperature of a sample) measured with a temperature sensor. In the graph illustrated in Fig. 9, a point indicated by M corresponds to a highest value of the sample temperature.

The calculation results for the maximum hygroscopic heat generation are shown in Table 7.

[Table 7]

| Raw material fiber | Maximum hygroscopic heat generation (° C/g) |
|---|---|
| PRT918 | 0.040 |
| PRT799 | 0.031 |
| Wool | 0.020 |
| Cotton | 0.021 |
| Tencel | 0.018 |
| Rayon | 0.025 |
| Polyester | 0.010 |

As shown in Table 7, it can be seen that the modified fibroin (PRT918 and PRT799) fibers have a high value of the maximum hygroscopic heat generation and excellent hygroscopic exothermicity, as compared with the existing materials. Accordingly, it can be said that the blended yarn containing such a modified fibroin fiber and a knitted or woven body of the same have excellent hygroscopic exothermicity.

<Reference Example 3>

A doping liquid of the modified fibroins PRT799 and PRT966 obtained in the same manner as in Example above was filtered through a metal filter having an opening of 5 μm at 60°C, then allowed to stand in a 30 mL stainless steel syringe, defoamed, and then discharged from a solid nozzle having a needle diameter of 0.2 mm into a 100%-by-mass methanol coagulation bath. The discharge temperature was 60°C. After coagulation, the obtained raw yarn was wound and naturally dried to obtain a modified fibroin fiber (raw material fiber).

For comparison, commercially available wool fibers, silk fibers, cotton fibers, rayon fibers, and polyester fibers were prepared as raw material fibers.

Each raw material fiber was used to manufacture a knitted fabric by flat knitting using a flat knitting machine. The count, the number of twists, the gauge number, and the basis weight of the knitted fabric using the PRT966 fiber and the PRT799 fiber are shown in Table 8. The thickness and the gauge number of the knitted fabric using other raw material fibers were adjusted so that the cover factor was almost the same as the cover factor of the knitted fabric of the modified fibroin fiber. Specifically, the values are as shown in Table 8.

[Table 8]

| Raw material fiber | Count [Nm] | Number of twists | Gauge number [GG] | Basis weight [g/m$^2$] |
|---|---|---|---|---|
| PRT966 | 30 | 1 | 18 | 90.1 |
| PRT799 | 30 | 1 | 16 | 111.0 |
| Wool | 30 | 2 | 14 | 242.6 |
| Silk | 60 | 2 | 14 | 225.2 |
| Cotton | 34 | 2 | 14 | 194.1 |
| Rayon | 38 | 1 | 14 | 181.8 |
| Polyester | 60 | 1 | 14 | 184.7 |

The heat retention was evaluated by a dry contact method, using a KES-F7 Thermo Labo II tester manufactured by Kato Tech Co., Ltd. The dry contact method is a method assuming a direct contact between the skin and clothes in a dry state. One sheet of the knitted fabric cut into a rectangle of 20 cm × 20 cm was used as a test piece (sample). The test piece was set on a hot plate set to a constant temperature (30°C), and the quantity of heat (a) dissipated through the test piece was determined under the conditions of a wind velocity in a wind tunnel of 30 cm/sec. The quantity of heat (b) dissipated through the test piece under the same conditions as above in a state where the test piece was not set was determined, and the heat retention rate (%) was calculated according to the following formula.

$$\text{Heat retention rate (\%)} = (1 - a/b) \times 100$$

From the measurement results, the heat retention index was calculated according to the following Formula B.

Heat retention index = Heat retention rate (%) / Basis weight (g/m$^2$) of sample                    Formula B:

The calculation results of the heat retention indices are shown in Table 9. The materials having a higher heat retention index can be evaluated to have more excellent heat retention.

[Table 9]

| Raw material fiber | Heat retention index |
|---|---|
| PRT966 | 0.33 |
| PRT799 | 0.22 |
| Wool | 0.16 |
| Silk | 0.11 |
| Cotton | 0.13 |
| Rayon | 0.02 |
| Polyester | 0.18 |

As shown in Table 9, it can be seen that the modified fibroin (PRT966 and PRT799) fibers have higher heat retention indices and excellent heat retention, as compared with the existing materials.

## Reference Signs List

1... extrusion device, 2... undrawn yarn manufacturing device, 3... wet heat drawing device, 4...drying device, 6...doping liquid, 10...spinning device, 20...coagulation liquid bath, 21...drawing bath, 36...modified fibroin filament.

## Claims

1. A blended yarn comprising:

   a modified fibroin fiber; and
   a regenerated cellulose fiber,
   wherein the modified fibroin fiber comprises a modified fibroin, and
   wherein the modified fibroin comprises a domain sequence represented by:

   (a)

   Formula 1:          $[(A)_n \text{ motif-REP}]_m$;

   or
   (b)

   Formula 2:          $[(A)_n \text{ motif-REP}]_m\text{-}(A)_n$ motif,

   wherein the $(A)_n$ motif represents an amino acid sequence composed of 2 to 27 amino acid residues and the number of alanine residues with respect to a total number of amino acid residues in the $(A)_n$ motif is 40% or more;
   REP represents an amino acid sequence composed of 2 to 200 amino acid residues;
   m represents an integer of 2 to 300;
   a plurality of the $(A)_n$ motifs may have the same amino acid sequence or amino acid sequences different from each other; and
   a plurality of REPs may have the same amino acid sequence or amino acid sequences different from each other.

2. The blended yarn according to claim 1, wherein a shrinkage rate of the modified fibroin fiber as defined by the following formula is more than 7%:

Shrinkage rate = {1 - (Length of modified fibroin fiber after --> being brought into contact with aqueous medium/ Length of modified fibroin fiber before being brought into contact with aqueous medium)} × 100 (%),

wherein the temperature of the aqueous medium is at least 10°C and at most 230°C, and
wherein the duration of contact with the aqueous medium is at least 30 seconds and at most 10 minutes.

3. The blended yarn according to claim 1 or 2, wherein an amount of the modified fibroin fiber is 5% to 40% by mass.

4. The blended yarn according to any one of claims 1 to 3, wherein the modified fibroin fiber is a modified spider silk fibroin fiber.

5. The blended yarn according to any one of claims 1 to 4, wherein the regenerated cellulose fiber is a lyocell fiber.

6. The blended yarn according to any one of claims 1 to 5, wherein

the modified fibroin comprises a domain sequence represented by Formula 1: $[(A)_n$ motif-REP$]_m$, and
the domain sequence has an amino acid sequence with a reduced content of glycine residues, which corresponds to an amino acid sequence in which at least one or a plurality of glycine residues in REP are substituted with other amino acid residues, as compared with a naturally occurring fibroin,
wherein in Formula 1, the number of alanine residues with respect to a total number of amino acid residues in the $(A)_n$ motif is 83% or more, and
REP represents an amino acid sequence composed of 10 to 200 amino acid residues.

7. The blended yarn according to any one of claims 1 to 5, wherein

the domain sequence has an amino acid sequence with a reduced content of glutamine residues, which corresponds to an amino acid sequence in which one or a plurality of glutamine residues in REP are deleted or substituted with other amino acid residues, as compared with a naturally occurring fibroin,
wherein the number of alanine residues with respect to a total number of amino acid residues in the $(A)_n$ motif is 80% or more, and
REP represents an amino acid sequence composed of 10 to 200 amino acid residues.

8. A knitted or woven body of the blended yarn according to any one of claims 1 to 7.

9. A method for manufacturing a knitted or woven body, comprising steps of:

blend-spinning a modified fibroin fiber and a regenerated cellulose fiber to obtain a blended yarn as claimed in any one of claims 1 to 7; and
knitting or weaving the blended yarn to obtain an unprocessed knitted or woven body.

10. The manufacturing method according to claim 9, wherein a shrinkage rate of the modified fibroin fiber as defined by the following formula is more than 7%:

Shrinkage rate = {1 - (Length of modified fibroin fiber after being brought into contact with aqueous medium/ Length of modified fibroin fiber before being brought into contact with aqueous medium)} × 100 (%),

wherein the temperature of the aqueous medium is at least 10°C and at most 230°C, and
wherein the duration of contact with the aqueous medium is at least 30 seconds and at most 10 minutes.

11. The manufacturing method according to claim 9 or 10, further comprising bringing the unprocessed knitted or woven body into contact with an aqueous medium to shrink the modified fibroin fiber contained in the unprocessed knitted or woven body.

12. The manufacturing method according to any one of claims 9 to 11, wherein in the step of obtaining the blended yarn, the modified fibroin fiber and the regenerated cellulose fiber are blend-spun so that an amount of the modified fibroin fiber in the blended yarn becomes 5% to 40% by mass.

13. The manufacturing method according to any one of claims 9 to 12, wherein the modified fibroin fiber is a modified spider silk fibroin fiber.

14. The manufacturing method according to any one of claims 9 to 13, wherein the regenerated cellulose fiber is a lyocell fiber.

**Patentansprüche**

1. Mischgarn, umfassend:

eine modifizierte Fibroinfaser, und
eine regenerierte Zellulosefaser,
wobei die modifizierte Fibroinfaser ein modifiziertes Fibroin umfasst, und
wobei das modifizierte Fibroin eine Domänensequenz umfasst, dargestellt durch:

(a)

Formel 1:          $[(A)_n \text{ Motiv-REP}]_m$,

oder
(b)

Formel 2:          $[(A)_n \text{ Motiv-REP}]_m\text{-}(A)_n\text{-Motiv}$,

wobei das $(A)_n$-Motiv eine aus 2 bis 27 Aminosäureresten zusammengesetzte Aminosäuresequenz darstellt, und wobei die Anzahl an Alaninresten in Bezug auf eine Gesamtanzahl an Aminosäureresten in dem $(A)_n$-Motiv 40 % oder mehr beträgt;
REP für eine aus 2 bis 200 Aminosäureresten zusammengesetzte Aminosäuresequenz steht;
m für eine ganze Zahl von 2 bis 300 steht;
eine Vielzahl von $(A)_n$-Motiven die gleiche Aminosäuresequenz oder voneinander unterschiedliche Aminosäuresequenzen aufweisen, und
eine Vielzahl von REPs die gleiche Aminosäuresequenz oder voneinander unterschiedliche Aminosäuresequenzen aufweisen können.

2. Mischgarn gemäß Anspruch 1, wobei eine Schrumpfungsrate der modifizierten Fibroinfaser, wie durch die folgende Formel definiert, mehr als 7 % beträgt:

Schrumpfungsrate = {1 - (Länge der modifizierten Fibroinfaser nach Inkontaktbringen mit wässrigem Medium/ Länge der modifizierten Fibroinfaser vor dem Inkontaktbringen mit wässrigem Medium)} x 100 (%),

wobei die Temperatur des wässrigen Mediums mindestens 10 °C und höchstens 230 °C beträgt, und
wobei die Dauer des Kontakts mit dem wässrigen Medium mindestens 30 Sekunden und höchstens 10 Minuten beträgt.

3. Mischgarn gemäß Anspruch 1 oder 2, wobei eine Menge der modifizierten Fibroinfaser 5 Massen-% bis 40 Massen-% beträgt.

**4.** Mischgarn gemäß einem der Ansprüche 1 bis 3, wobei die modifizierte Fibroinfaser eine modifizierte Spinnenseidenfibroinfaser ist.

**5.** Mischgarn gemäß einem der Ansprüche 1 bis 4, wobei die regenerierte Zellulosefaser eine Lyocellfaser ist.

**6.** Mischgarn gemäß einem der Ansprüche 1 bis 5, wobei

das modifizierte Fibroin eine Domänensequenz umfasst, dargestellt durch Formel 1: $[(A)_n \text{ Motiv-REP}]_m$, und die Domänensequenz eine Aminosäuresequenz mit einem reduzierten Gehalt an Glycinresten aufweist, die einer Aminosäuresequenz entspricht, bei der mindestens ein oder eine Vielzahl von Glycinresten in REP im Vergleich zu einem natürlich vorkommenden Fibroin durch andere Aminosäurereste substituiert ist, wobei in Formel 1 die Anzahl an Alaninresten in Bezug auf eine Gesamtanzahl an Aminosäureresten in dem $(A)_n$-Motiv 83 % oder mehr beträgt, und REP für eine aus 10 bis 200 Aminosäureresten zusammengesetzte Aminosäuresequenz steht.

**7.** Mischgarn gemäß einem der Ansprüche 1 bis 5, wobei die Domänensequenz eine Aminosäuresequenz mit einem reduzierten Gehalt an Glutaminresten aufweist, die einer Aminosäuresequenz entspricht, bei der ein oder eine Vielzahl von Glutaminresten in REP im Vergleich zu einem natürlich vorkommenden Fibroin deletiert oder durch andere Aminosäurereste substituiert ist,

wobei die Anzahl an Alaninresten in Bezug auf eine Gesamtanzahl an Aminosäureresten in dem $(A)_n$-Motiv 80 % oder mehr beträgt, und REP für eine aus 10 bis 200 Aminosäureresten zusammengesetzte Aminosäuresequenz steht.

**8.** Gestrickter oder gewebter Körper des Mischgarns gemäß einem der Ansprüche 1 bis 7.

**9.** Verfahren zum Fertigen eines gewirkten oder gewebten Körpers, umfassend folgende Schritte:

Mischspinnen einer modifizierten Fibroinfaser und einer regenerierten Zellulosefaser, um ein Mischgarn gemäß einem der Ansprüche 1 bis 7 zu erhalten, und Stricken oder Weben des Mischgarns, um einen unverarbeiteten gestrickten oder gewebten Körper zu erhalten.

**10.** Fertigungsverfahren gemäß Anspruch 9, wobei eine Schrumpfungsrate der modifizierten Fibroinfaser, wie durch die folgende Formel definiert, mehr als 7 % beträgt:

Schrumpfungsrate = {1 - (Länge der modifizierten Fibroinfaser nach dem Inkontaktbringen mit wässrigem Medium/Länge der modifizierten Fibroinfaser vor dem Inkontaktbringen mit wässrigem Medium)} x 100 (%),

wobei die Temperatur des wässrigen Mediums mindestens 10 °C und höchstens 230 °C beträgt, und wobei die Dauer des Kontakts mit dem wässrigen Medium mindestens 30 Sekunden und höchstens 10 Minuten beträgt.

**11.** Fertigungsverfahren gemäß Anspruch 9 oder 10, ferner umfassend das Inkontaktbringen des unverarbeiteten gewirkten oder gewebten Körpers mit einem wässrigen Medium, um die in dem unverarbeiteten gewirkten oder gewebten Körper enthaltene modifizierte Fibroinfaser zu schrumpfen.

**12.** Fertigungsverfahren gemäß einem der Ansprüche 9 bis 11, wobei in dem Schritt des Erhaltens des Mischgarns die modifizierte Fibroinfaser und die regenerierte Zellulosefaser so mischgesponnen werden, dass eine Menge der modifizierten Fibroinfaser in dem Mischgarn 5 Massen-% bis 40 Massen- % beträgt.

**13.** Fertigungsverfahren gemäß einem der Ansprüche 9 bis 12, wobei die modifizierte Fibroinfaser eine modifizierte Spinnenseidenfibroinfaser ist.

**14.** Fertigungsverfahren gemäß einem der Ansprüche 9 bis 13, wobei die regenerierte Zellulosefaser eine Lyocellfaser ist.

**Revendications**

1. Fil mélangé comprenant :

    une fibre de fibroïne modifiée ; et
    une fibre de cellulose régénérée,
    dans lequel la fibre de fibroïne modifiée comprend une fibroïne modifiée, et
    dans lequel la fibroïne modifiée comprend une séquence de domaine représentée par :

    (a)

    Formule 1 :        [motif $(A)_n$-REP]$_m$ ;

    ou
    (b)

    Formule 2 :        [motif $(A)_n$-REP]$_m$-motif $(A)_n$,

    dans lequel le motif $(A)_n$ représente une séquence d'acides aminés composée de 2 à 27 résidus d'acides aminés et le nombre de résidus d'alanine par rapport à un nombre total de résidus d'acides aminés dans le motif $(A)_n$ est de 40 % ou plus ;
    REP représente une séquence d'acides aminés constituée de 2 à 200 résidus d'acides aminés ;
    m représente un nombre entier de 2 à 300 ;
    une pluralité des motifs $(A)_n$ peuvent avoir la même séquence d'acides aminés ou des séquences d'acides aminés différentes les unes des autres ; et
    une pluralité de REP peuvent avoir la même séquence d'acides aminés ou des séquences d'acides aminés différentes les unes des autres.

2. Fil mélangé selon la revendication 1, dans lequel un taux de retrait de la fibre de fibroïne modifiée tel que défini par la formule suivante est supérieur à 7 % :

    Taux de retrait = {1 - (Longueur de la fibre de fibroïne modifiée après avoir été mise en contact avec un milieu aqueux/Longueur de fibre de fibroïne modifiée avant d'être mise en contact avec un milieu aqueux)} x 100 (%),

    dans lequel la température du milieu aqueux est d'au moins 10° C et d'au plus 230° C, et
    dans lequel la durée de contact avec le milieu aqueux est d'au moins 30 secondes et d'au plus 10 minutes.

3. Fil mélangé selon la revendication 1 ou 2, dans lequel une quantité de la fibre de fibroïne modifiée est de 5 % à 40 % en masse.

4. Fil mélangé selon l'une quelconque des revendications 1 à 3, dans lequel la fibre de fibroïne modifiée est une fibre de fibroïne de soie d'araignée modifiée.

5. Fil mélangé selon l'une quelconque des revendications 1 à 4, dans lequel la fibre de cellulose régénérée est une fibre de lyocell.

6. Fil mélangé selon l'une quelconque des revendications 1 à 5, dans lequel

    la fibroïne modifiée comprend une séquence de domaine représentée par la formule 1 : [motif $(A)_n$-REP]$_m$, et
    la séquence de domaine comporte une séquence d'acides aminés avec une teneur réduite en résidus de glycine, qui correspond à une séquence d'acides aminés dans laquelle au moins un ou une pluralité de résidus de glycine dans REP sont substitués par d'autres résidus d'acides aminés, par rapport à une fibroïne d'origine naturelle,
    dans lequel, dans la formule 1, le nombre de résidus d'alanine par rapport à un nombre total de résidus d'acides aminés dans le motif $(A)_n$ est de 83 % ou plus, et

REP représente une séquence d'acides aminés constituée de 10 à 200 résidus d'acides aminés.

7. Fil mélangé selon l'une quelconque des revendications 1 à 5, dans lequel

la séquence de domaine comporte une séquence d'acides aminés avec une teneur réduite en résidus de glutamine, qui correspond à une séquence d'acides aminés dans laquelle un ou une pluralité de résidus de glutamine dans REP sont supprimés ou substitués par d'autres résidus d'acides aminés, par rapport à une fibroïne d'origine naturelle,
dans lequel le nombre de résidus d'alanine par rapport à un nombre total de résidus d'acides aminés dans le motif $(A)_n$ est de 80 % ou plus, et
REP représente une séquence d'acides aminés constituée de 10 à 200 résidus d'acides aminés.

8. Corps tricoté ou tissé du fil mélangé selon l'une quelconque des revendications 1 à 7.

9. Procédé permettant la fabrication d'un corps tricoté ou tissé, comprenant les étapes de :

filage en mélange d'une fibre de fibroïne modifiée et d'une fibre de cellulose régénérée pour obtenir un fil mélangé selon l'une quelconque des revendications 1 à 7 ; et
tricotage ou tissage du fil mélangé pour obtenir un corps tricoté ou tissé non traité.

10. Procédé de fabrication selon la revendication 9, dans lequel un taux de retrait de la fibre de fibroïne modifiée tel que défini par la formule suivante est supérieur à 7 % :

Taux de retrait = {1 - (Longueur de fibre de fibroïne modifiée après avoir été mise en contact avec un milieu aqueux/Longueur de fibre de fibroïne modifiée avant d'être mise en contact avec un milieu aqueux)} x 100 (%),

dans lequel la température du milieu aqueux est d'au moins 10° C et d'au plus 230° C, et
dans lequel la durée de contact avec le milieu aqueux est d'au moins 30 secondes et d'au plus 10 minutes.

11. Procédé de fabrication selon la revendication 9 ou 10, comprenant en outre la mise en contact du corps tricoté ou tissé non traité avec un milieu aqueux pour rétracter la fibre de fibroïne modifiée contenue dans le corps tricoté ou tissé non traité.

12. Procédé de fabrication selon l'une quelconque des revendications 9 à 11, dans lequel dans l'étape d'obtention du fil mélangé, la fibre de fibroïne modifiée et la fibre de cellulose régénérée sont filées en mélange de sorte qu'une quantité de la fibre de fibroïne modifiée dans le fil mélangé devienne de 5 % à 40 % en masse.

13. Procédé de fabrication selon l'une quelconque des revendications 9 à 12, dans lequel la fibre de fibroïne modifiée est une fibre de fibroïne de soie d'araignée modifiée.

14. Procédé de fabrication selon l'une quelconque des revendications 9 à 13, dans lequel la fibre de cellulose régénérée est une fibre de lyocell.

# Fig.1

EP 3 808 882 B1

**Fig.2**

EP 3 808 882 B1

## Fig.3

Fig.4

# Fig.5

**Fig.6**

EP 3 808 882 B1

*Fig.7*

Fig.8

**Fig.9**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 6162566 B
- CN 104178898
- CN 103349383
- WO 2006008163 A
- CN 103132198 A
- WO 2012165477 A
- JP 2002238569 A

**Non-patent literature cited in the description**

- **MITTAL et al.** *ACS Nano*, 2017, vol. 11, 5148-5159
- Test Method for Granular or Low-Melting-Point Synthetic Resin. *Notice No. 50 of the Office of Hazardous Materials Regulation*, 31 May 1995
- *Nucleic Acid Res.*, 1982, vol. 10, 6487
- *Methods in Enzymology*, 1983, vol. 100, 448
- **KYTE J** ; **DOOLITTLE R**. A simple method for displaying the hydropathic character of a protein. *J. Mol. Biol.*, 1982, vol. 157, 105-132
- *Proc. Natl. Acad. Sci. USA*, 1972, vol. 69, 2110
- Molecular Cloning